(19) Europäisches Patentamt
European Patent Office
Office européen des brevets

(11) EP 2 311 834 A1

(12) EUROPEAN PATENT APPLICATION
published in accordance with Art. 153(4) EPC

(43) Date of publication:
**20.04.2011 Bulletin 2011/16**

(21) Application number: **09803011.7**

(22) Date of filing: **30.07.2009**

(51) Int Cl.:
***C07D 417/12*** *(2006.01)* ***A61K 31/427*** *(2006.01)*
***A61P 3/10*** *(2006.01)* ***A61P 35/00*** *(2006.01)*
***A61P 43/00*** *(2006.01)* ***C07B 63/00*** *(2006.01)*

(86) International application number:
**PCT/JP2009/063558**

(87) International publication number:
**WO 2010/013769 (04.02.2010 Gazette 2010/05)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **31.07.2008 JP 2008197307**

(71) Applicant: **Daiichi Sankyo Company, Limited**
**Chuo-ku**
**Tokyo 103-8426 (JP)**

(72) Inventors:
- **KAJINO, Hisaki**
  **Kanagawa 254-0014 (JP)**
- **NUMAGAMI, Eiji**
  **Kanagawa 254-0014 (JP)**
- **NIHEI, Satoru**
  **Kanagawa 254-0014 (JP)**

(74) Representative: **Fairbairn, Angus Chisholm**
**Marks & Clerk LLP**
**90 Long Acre**
**London**
**WC2E 9RA (GB)**

# (54) CRYSTAL OF THIAZOLIDINEDIONE COMPOUND, AND PROCESS FOR PRODUCTION THEREOF

(57) Thiazolidinedione compound crystalline forms useful as a bulk material for the manufacture of a peroxisome proliferator-activated receptor (PPAR) γ activator or an anticancer pharmaceutical composition are provided, which are hydrate crystalline forms of 5-(4-{[6-(4-amino-3,5-dimethylphenoxy)-l-methyl-l-H-benzimi dazol-2-yl]methoxylbenzyl)-l, 3-thiazolidine-2,4-dione represented by Formula (I) below and of its dihydrochloride.

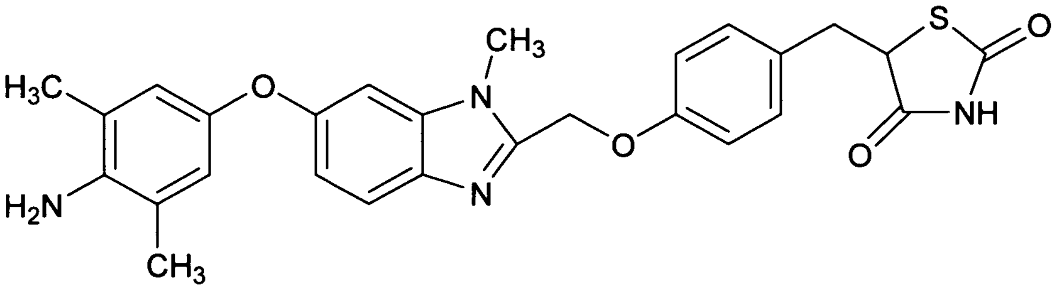

(I)

EP 2 311 834 A1

## Description

### Technical Field

The present invention relates to crystalline forms of a thiazolidinedione compound having a significant ability to activate peroxisome proliferator-activated receptor (PPAR) γ, a significant anticancer activity, advantageous physical properties for manufacturing pharmaceutical preparations, high purity, and significant storage stability and easy handling ability, and also relates to methods for their manufacture and to medicaments (specifically, PPAR γ activators or anticancer pharmaceutical compositions) containing the crystalline form(s) of the thiazolidinedione compound as an active ingredient.

### Background Art

Japanese Patent No. 3488099 (International Publication No. WO 99/18081, U.S. Patent No. 6432993, European Patent No. 1022272) (Patent Document 1), Japanese Patent Application Laid-Open (JP-A) No. 2003-238406 (WO 03/053440) (Patent Document 2), JP-A No. 2004-083574 (WO 2004/000356) (Patent Document 3), JP-A No. 2005-162727 (WO 2004/083167) (Patent Document 4), and WO 2007/091622 (Patent Document 5) disclose a thiazolidinedione compound represented by Formula (I) shown below (hereinafter referred to as Compound (I)). Compound (I) exhibits a significant ability to activate peroxisome proliferator-activated receptor (PPAR) γ and is expected to be useful for a PPAR γ activator or an anticancer pharmaceutical composition.
In general, pharmaceutical materials are required to have high purity so as not to cause unexpected, impurity-induced side effects. Impurities include by-products (analog substances) produced during the manufacture of the bulk pharmaceutical materials themselves, raw materials and solvents used in the manufacture of the bulk pharmaceutical materials, and the like. Pharmaceutical materials are also required to have more advantageous physicochemical properties for the manufacture of pharmaceutical preparations, such as that the crystalline form of the bulkmaterials does not change even during a heat treatment process or other processes used for preparation, or that they have high solubility so that they can be highly absorbable and effective when administered at low doses. It is also important that the bulk pharmaceutical materials can be stored for long periods of time whilst maintaining their quality. If they are required to be stored at low temperatures, large-scale refrigerating systems will be necessary for maintaining the quality. Therefore, it is industrially significant to find stable crystalline forms capable of being stored at room temperature or higher temperatures.
As suggested above, there is also a demand for methods of manufacturing bulk pharmaceutical materials industrially and on a large scale, having higher purity and advantageous physical properties for the manufacture of pharmaceutical preparations and capable of being stored for long periods of time whilst maintaining their purity and physical properties.
For commercialization of Compound (I) as a bulk pharmaceutical material, therefore, there is a need for advantageous physical properties for the manufacture of pharmaceutical preparations as mentioned above, higher purity and storage stability, and easy handling ability.

### Prior Art Documents

Patent Document 1: Japanese Patent No. 3488099 (International Publication No. WO 99/18081, U.S. Patent No. 6432993, European Patent No. 1022272)
Patent Document 2: Japanese Patent Application Laid-Open (JP-A) No. 2003-238406 (WO 03/053440)
Patent Document 3: JP-A No. 2004-083574 (WO 2004/000356)
Patent Document 4: JP-A No. 2005-162727 (WO 2004/083167)
Patent Document 5: WO 2007/091622

### Summary of the Invention

### Problems to be Solved by the Invention

The present inventors carried out research on Compound (I) which exhibits a significant ability to activate peroxisome proliferator-activated receptor (PPAR) γ and is expected to be useful for a PPAR γ activator or an anticancer pharmaceutical composition. As a result, the inventors have found novel hydrate crystalline forms of Compound (I) and its dihydrochloride, which have advantageous physicochemical properties as bulk pharmaceutical materials, high storage

stability at room temperature, and high purity, and methods for their manufacture, and thus have completed the invention.

Means for Solving the Problems

Thus, the invention is directed to:

(1) A hydrate crystalline form of 5-(4-{[6-(4-amino-3,5-dimethylphenoxy)-1-methyl-1-H-benzimi dazol-2-yl]methoxylbenzyl)-1,3-thiazolidine-2,4-dione represented by Formula (I):

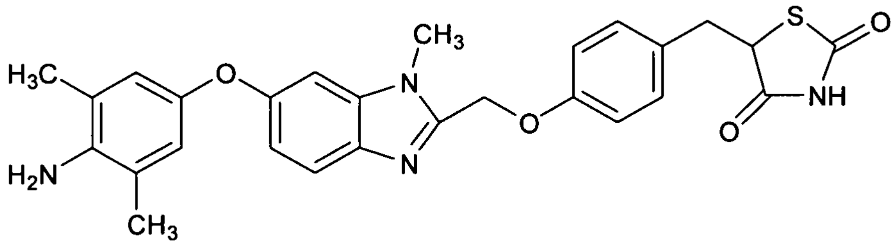

(I)

(2) A monohydrate crystalline form of 5-(4-{[6-(4-amino-3,5-dimethylphenoxy)-l-methyl-l-H-benzimi dazol-2-yl]methoxy}benzyl)-1,3-thiazolidine-2,4-dione represented by Formula (I) shown in Item (1), which shows peaks at interplanar spacings of 5.81, 5.60, 4.44, 4.16, and 3.70 angstroms in X-ray powder diffraction obtained using copper Kα radiation with a wavelength λ of 1.54 angstroms;

(3) A monohydrate crystalline form of the dihydrochloride of 5-(4-{[6-(4-amino-3,5-dimethylphenoxy)-l-methyl-l-H-benzimi dazol-2-yl]methoxy}benzyl)-1,3-thiazolidine-2,4-dione represented by Formula (I) shown in Item (1), which shows peaks at interplanar spacings of 10.93, 7.16, 5.84, 4.41, 3.65, and 3.49 angstroms in X-ray powder diffraction obtained using copper Kα radiation with a wavelength λ of 1.54 angstroms;

(4) A monohydrate crystalline form of the dihydrochloride of 5-(4-{[6-(4-amino-3,5-dimethylphenoxy)-l-methyl-l-H-benzimi dazol-2-yl]methoxy}benzyl)-1,3-thiazolidine-2,4-dione represented by Formula (I) shown in Item (1), which shows peaks at interplanar spacings of 10.88, 7.12, 5.82, 3.68, 3.49, 3.01, and 2.97 angstroms in X-ray powder diffraction obtained using copper Kα radiation with a wavelength λ of 1.54 angstroms;

(5) A pharmaceutical composition containing as an active ingredient the crystalline form of the thiazolidinedione compound represented by Formula (I) according to any one selected from Items (1) to (4);

(6) A PPAR γ activator containing as an active ingredient the crystalline form of the thiazolidinedione compound represented by Formula (I) according to any one selected from Items (1) to (4);

(7) An anticancer pharmaceutical composition containing as an active ingredient the crystalline form of the thiazolidinedione compound represented by Formula (I) according to any one selected from Items (1) to (4);

(8) A pharmaceutical composition for preventing or treating diabetes, containing as an active ingredient the crystalline form of the thiazolidinedione compound represented by Formula (I) according to any one selected from Items (1) to (4);

(9) A pharmaceutical composition for preventing or treating cancer occurring with type 2 diabetes, containing as an active ingredient the crystalline form of the thiazolidinedione compound represented by Formula (I) according to any one selected from Items (1) to (4);

(10) A method of manufacturing the crystalline form according to Item (2), characterized by desalting (neutralizing) a salt of 5-(4-{[6-(4-amino-3,5-dimethylphenoxy)-l-methyl-l-H-benzimi dazol-2-yl]methoxylbenzyl)-1,3-thiazolidine-2,4-dione or a hydrate thereof in a water-containing solvent;

(11) A method of manufacturing the crystalline form according to Item (3), characterized by: preparing a solution or suspension of 5-(4-{[6-(4-amino-3,5-dimethylphenoxy)-l-methyl-l-H-benzimi dazol-2-yl]methoxy}benzyl)-1,3-thiazolidine-2,4-dione in aqueous tetrahydrofuran; and then adding hydrogen chloride or hydrochloric acid to the resulting solution or suspension;

(12) A method of manufacturing the crystalline form according to Item (4), characterized by: dissolving 5-(4-{[6-(4-amino-3,5-dimethylphenoxy)-l-methyl-l-H-benzimi dazol-2-yl]methoxy}benzyl)-1,3-thiazolidine-2,4-dione in a tetrahydrofuran-free, aqueous solution; and then adding hydrogen chloride or hydrochloric acid to the resulting solution;

(13) A method of manufacturing the crystalline form according to Item (4), characterized by adding hydrogen chloride or hydrochloric acid to a suspension of a hydrate crystalline form of 5-(4-{[6-(4-amino-3,5-dimethylphenoxy)-l-methyl-l-H-benzimi dazol-2-yl]methoxy}benzyl)-1,3-thiazolidine-2,4-dione.

In an embodiment of the invention, the hydrate crystalline form of 5-(4-{[6-(4-amino-3,5-dimethylphenoxy)-1-methyl-1-H-benzimi dazol-2-yl]methoxy}benzyl)-1,3-thiazolidine-2,4-dione (hereinafter also referred to as Compound (I)) and the hydrate crystalline form of 5-(4-{[6-(4-amino-3,5-dimethylphenoxy)-1-methyl-1-H-benzimi dazol-2-yl]methoxy}benzyl)-1,3-thiazolidine-2,4-dione dihydrochloride (hereinafter each also referred to as the crystalline form of the invention) are solids having an internal structure including three-dimensionally, regularly repeating constituent atoms (or groups thereof) and distinguished from an amorphous solid not having such a regular internal structure.

Even a single compound can form two or more different crystalline forms having different internal structures and physicochemical properties (crystal polymorphs), depending on crystallization conditions. The crystalline form of the invention may be any of such crystal polymorphs or a mixture of two or more different crystal polymorphs.

When allowed to stand in the air, the crystalline form of the invention may absorb water and carry adherent water, or when mixed with an organic solvent, the crystalline form of the invention may absorb the solvent to form a solvate. The crystalline form of the invention may also form a 0.5 hydrate or an anhydride when subjected to heating at 25 to 150°C under normal atmospheric conditions or other treatments.

The crystalline form of the invention is intended to include such adherent water-containing crystalline forms, hydrated or solvated crystalline forms, 0.5 hydrate-containing crystalline forms, or anhydride-containing crystalline forms. The crystalline form of the invention is preferably a monohydrate crystalline form of Compound (I) or a monohydrate crystalline form of the dihydrochloride of Compound (I).

In an embodiment of the invention, the monohydrate crystalline form of Compound (I) is typically a crystalline form that shows peaks at interplanar spacings of 5.81, 5.60, 4.44, 4.16, and 3.70 angstroms in X-ray powder diffraction obtained using copper K$\alpha$ radiation with a wavelength $\lambda$ of 1.54 angstroms (hereinafter also referred to as "free-form crystalline form ").

In an embodiment of the invention, the monohydrate crystalline form of the dihydrochloride of Compound (I) is typically a crystalline form that shows peaks at interplanar spacings of 10.93, 7.16, 5.84, 4.41, 3.65, and 3.49 angstroms in X-ray powder diffraction obtained using copper K$\alpha$ radiation with a wavelength $\lambda$ of 1. 54 angstroms (hereinafter also referred to as " (Crystalline form A") or a crystalline form that shows peaks at interplanar spacings of 10.88, 7.12, 5.82, 3.68, 3.49, 3.01, and 2.97 angstroms in X-ray powder diffraction obtained using copper K$\alpha$ radiation with a wavelength $\lambda$ of 1.54 angstroms (hereinafter also referred to as "Crystalline form B").

The interplanar spacing d may be calculated from the formula: $2d \cdot \sin\theta = n\lambda$, in which n = 1.

The relative intensity of the peaks may vary with crystal growth face or the like (crystal habit). Even in such cases, crystals having the same form are also covered by the scope of the invention.

Effects of the Invention

The crystalline form of the invention has a high purity, a white color tone, and a high level of storage stability and easy handling ability.

In particular, the crystalline form of a monohydrate of Compound (I) according to the invention (free-form crystalline form) has a higher solubility in dilute hydrochloric acid than known conventional salts of Compound (I). Crystalline form B, a dihydrochloride monohydrate of Compound (I) according to the invention has a low residual solvent content, a high purity, a white color tone, and a high hydrate-dehydration temperature, and therefore, the hydrate has high storage stability at room temperature. Crystalline form A, a dihydrochloride monohydrate of Compound (I) according to the invention has a higher solubility than Crystalline form B.

Therefore, the hydrate and dihydrochloride hydrate crystalline forms of Compound (I) according to the invention are useful as bulk pharmaceutical materials for the manufacture of medicines (specifically, PPAR $\gamma$ activators, agents for preventing and/or treating cancers, agents for preventing and/or treating diabetes, or agents for preventing and/or treating cancers occurring with type 2 diabetes) to be industrially prepared on a large scale.

The crystal manufacturing method of the invention makes it possible to manufacture crystalline forms having a high purity and higher solubility in dilute hydrochloric acid or a lower residual solvent content, and is therefore useful for manufacturing bulk pharmaceutical materials for medicines to be industrially prepared on a large scale.

Brief Description of Drawings

Fig. 1 is an X-ray powder diffraction pattern of Crystalline form A obtained in Example 1, in which the vertical axis represents diffraction intensity in units of counts/second (cps), and the horizontal axis represents diffraction angle 2$\theta$ value;

Fig. 2 is an X-ray powder diffraction pattern of Crystalline form B obtained in Example 2, in which the vertical axis represents diffraction intensity in units of counts/second (cps), and the horizontal axis represents diffraction angle 2$\theta$ value;

Fig. 3 is an X-ray powder diffraction pattern of the free-form crystalline form obtained in Example 3, in which the vertical axis represents diffraction intensity in units of counts/second (cps), and the horizontal axis represents diffraction angle 2θ value;

Fig. 4 is an X-ray powder diffraction pattern of the compound obtained in Comparative Example 1, in which the vertical axis represents diffraction intensity in units of counts/second (cps), and the horizontal axis represents diffraction angle 2θ value; and

Fig. 5 is an X-ray powder diffraction pattern of the crystalline form obtained in Example (2-1) of Example 2, in which the vertical axis represents diffraction intensity in units of counts/second (cps), and the horizontal axis represents diffraction angle 2θ value.

Embodiments for Carrying out the Invention

Compound (I) described above can be manufactured by the method disclosed in Japanese Patent No. 3488099 or methods analogous thereto.

The hydrate crystalline forms of Compound (I) according to the invention may be manufactured by a process including desalting (neutralizing) a solution or suspension of Compound (I) or a salt thereof or a hydrate thereof in water or a water-containing solvent, bringing the solution or suspension into a supersaturated state by concentration, cooling, mixing of good and poor solvents, or any other method to precipitate hydrate crystals of Compound (I), and isolating the precipitated crystals.

In an embodiment of the invention, examples of a salt of Compound (I) that may be used include a metal salt such as an alkali metal salt such as a sodium or potassium salt, an alkaline-earth metal salt such as a calcium or magnesium salt, or an aluminum salt; an amine salt such as an inorganic salt such as an ammonium salt or an organic salt such as an ethylenediamine salt, a guanidine salt, or a diethylamine salt; a halogenated hydroacid salt such as a hydrofluoric acid salt, a hydrochloride, or a hydrobromic acid salt; an inorganic acid salt such as a nitrate, a perchlorate, a sulfate, or a phosphate; an organic acid salt such as a lower alkanesulfonate such as a methanesulfonate, a trifluoromethanesulfonate, or an ethanesulfonate, an arylsulfonate such as a benzenesulfonate, an acetate, a malate, a fumarate, a succinate, a citrate, a tartrate, an oxalate, or a maleate; an amino acid salt such as a glutamate or an aspartate; and various other salts.

In an embodiment of the invention, a solution or suspension of Compound (I) or a salt thereof or a hydrate thereof in water or a water-containing solvent may be used as a starting material for the manufacture of the hydrate crystalline form of Compound (I). For example, the solution or suspension to be used may also be a solution or suspension of a Compound (I)-containing crude product of a synthetic reaction, so that such a solution or suspension can be purified by crystallization.

The manufacture of crystalline forms having a hydrate structure requires water and, therefore, a water-miscible solvent is used in the manufacture of the hydrate crystalline form of Compound (I) according to the invention.

For example, such a water-miscible solvent may be a lower alcohol such as methanol, ethanol or propanol, tetrahydrofuran, or dimethylformamide, or a mixed solvent thereof, preferably a lower alcohol, more preferably methanol.

In an embodiment of the invention, an acid or a base may be used in the process of desalting a salt of Compound (I) or various solvates thereof. The acid to be used may be any acid with which the desalting is possible. For example, the acid to be used may be any of various acids such as hydrogen halides such as hydrogen fluoride, hydrogen chloride and hydrogen bromide; inorganic acids such as nitric acid, perchloric acid, sulfuric acid, and phosphoric acid; organic acids such as lower alkanesulfonic acids such as methanesulfonic acid, trifluoromethanesulfonic acid and ethanesulfonic acid, arylsulfonic acids suchasbenzenesulfonicacid, and acetic acid, malic acid, fumaric acid, succinic acid, citric acid, tartaric acid, oxalic acid, and maleic acid; and amino acids such as glutamic acid and aspartic acid, preferably a hydrogen halide or an inorganic acid, more preferably hydrogen chloride, hydrogen bromide or sulfuric acid. The base to be used may be any base with which the desalting is possible. For example, the base to be used may be an alkali metal hydroxide such as lithium hydroxide, sodium hydroxide, potassium hydroxide, or cesium hydroxide, an alkaline-earth metal hydroxide such as magnesium hydroxide or calcium hydroxide, an alkaline-earth metal oxide such as magnesium oxide or calcium oxide, or ammonia or an amine such asmethylamine, dimethylamine, trimethylamine, ethylamine, diethylamine, triethylamine, or tributylamine, preferably an alkali metal hydroxide or an amine, more preferably lithium hydroxide, sodium hydroxide, potassium hydroxide, ammonia, or tributylamine.

The acid or the base may be added as it is or in the form of a solution in any of various solvents.

The acid or the base necessary for the desalting may be added in any amount, depending on the amount of the solvent used and the desalting temperature. While the amount of the acid or the base to be used may be any such that the solubility of the hydrate crystalline form of Compound (I) according to the invention can be mostly minimized under the desalting conditions, it is generally 0 to 100 equivalents, preferably 0 to 10 equivalents, more preferably 0 to 5 equivalents, based on the amount of any of various salts of Compound (I).

The amount of the solvent used in the desalting is preferably, but not limited to, at least one part by weight,

more preferably at least five parts by weight, even more preferably at least 10 parts by weight, based on one part by weight of any of various salts of Compound (I) used, depending on the amount of the acid or the base used and the desalting temperature.

The desalting temperature is generally, but not limited to, 0°C or more, preferably 10°C or more, more preferably 30°C or more, depending on the amount of the solvent or the base used.

After the desalting (neutralization), the Compound (I) hydrate solution is brought into a supersaturated state so that the hydrate of Compound (I) can be precipitated. As mentioned above, concentrating the solution is a method of achieving supersaturation. For example, such a method may be a method of concentrating the solution of Compound (I) by evaporating the solvent by heating under normal or reduced pressure with a rotary evaporator or the like, or a method of concentrating the solution of Compound (I) with a reverse osmosis membrane. For example, the reverse osmosis membrane for use in the concentration of the solution may be selected from a polyacrylonitrile membrane, a polyvinyl alcohol membrane, a polyamide membrane, a cellulose acetate membrane, and the like.

The temperature for the crystallization of the hydrate crystalline form of Compound (I) according to the invention is generally from -70 to 150°C, preferably from -70 to 100°C.

While crystals may spontaneously start to precipitate in the reaction vessel, precipitation of the crystals may be started or promoted by adding a seed crystal or applying ultrasonic stimulation or mechanical stimulation such as by scratching the surface of the reaction vessel.

When a seed crystal is used, the seed crystal is not particularly restricted as long as it is a crystalline form of Compound (I) according to the invention. In a preferred mode, it is the free-form crystalline form according to the invention.

The amount of the seed crystal is preferably, but not limited to, 0.01 to 20% by weight, more preferably 0.1 to 10% by weight, based on the weight of Compound (I) or the salt of Compound (I) used or any of various solvates thereof.

The precipitated hydrate crystalline form of Compound (I) according to the invention may typically be isolated by filtration, centrifugation, decantation, or any other technique.

The isolated hydrate crystalline form of Compound (I) according to the invention may be washed with an appropriate solvent, if necessary. Washing the crystalline form of the invention may be performed, for example, using water, a lower alkyl alcohol such as methanol, ethanol or propanol, a lower alkylnitrile such as acetonitrile or propionitrile, tetrahydrofuran, dimethylformamide, or a mixed solvent thereof, preferably using water.

The purity and quality of the resulting crystalline form may be improved by recrystallization or slurry purification.

Crystalline formA of the invention, which is a monohydrate crystalline form of the dihydrochloride of Compound (I), may be manufactured by a process including: dissolving Compound (I) or a salt of Compound (I) or any of various solvates thereof, the dihydrochloride of Compound (I) or any of various solvates thereof, or the Compound (I) dihydrochloride hydrate itself in aqueous tetrahydrofuran; subjecting the solution to desalting (neutralization) ; adding hydrogen chloride or hydrochloric acid to the solution; bringing the solution into a supersaturated state by concentrating or cooling the solution or mixing of good and poor solvents or any other method to precipitate a dihydrochloride hydrate of Compound (I); and isolating the precipitated crystalline form.

The already isolated Compound (I) may be used as a starting material for the manufacture of Crystalline form A. Alternatively, a solution or suspension of a Compound (I)-containing crude product of a synthetic reaction performed in tetrahydrofuran may be used, or a solution or suspension obtained by desalting (neutralizing) a salt of Compound (I) or any of various solvates thereof in aqueous tetrahydrofuran may be used, because Compound (I) in such a solution or suspension can be purified by the crystallization.

Aqueous tetrahydrofuran is used in the manufacture of Crystalline form A.

The water content of aqueous tetrahydrofuran is generally 1 to 50%, preferably 2 to 40%, although it may be at any level equal to or more than the amount with which hydration is possible.

When a salt of Compound (I) or any of various solvates thereof is used as a starting material for the manufacture of Crystalline formA, desalting (neutralization) may be performed to ensure the production of the dihydrochloride of Compound (I). In such a case, an acid or a base is generally used for the desalting. The acid or base to be used may be any acid or base with which the desalting is possible. Examples of such an acid or base that may be used include the acids or bases listed above for use in the desalting for the manufacture of the hydrate crystalline form of Compound (I) according to the invention. Such an acid or base is preferably a hydrogen halide, an inorganic acid, an alkali metal hydroxide, or an amine, more preferably hydrogen chloride, hydrogen bromide, sulfuric acid, lithium hydroxide, sodium hydroxide, potassium hydroxide, ammonia, or tributylamine.

The acid or the base may be added as it is or in the form of a solution in any of various solvents.

The amount of the acid or base to be added is generally, but not limited to, 0.1 to 4 equivalents, based on the amount of any of various salts of Compound (I).

Crystalline form A may also be manufactured by adding hydrogen chloride or hydrochloric acid to a solution or suspension of the free-form crystalline form of the invention.

The suspension of the free-form crystalline form of the invention to be used may be prepared by suspending the isolated free-form crystalline form of the invention in water. Alternatively, a suspension obtained by desalting (neutralizing) a salt of Compound (I) or any of various solvates thereof may also be used. Such a suspension may be manufactured according to the method of manufacturing the free-form crystalline form of the invention.

When hydrogen chloride or hydrochloric acid is added for the crystallization of Crystalline form A, the amount of the addition is preferably equal to or more than the amount necessary for the formation of the dihydrochloride of Compound (I) and for the crystallization thereof. In general, it may also be added in an amount of 0.1 to 20 equivalents, preferably 2 to 10 equivalents, based on the amount of Compound (I), so that the solubility of the dihydrochloride hydrate of Compound (I) in the solvent can be reduced.

Hydrogen chloride may be added directly or in the form of a solution in any of various organic solvents. Such organic solvents may be any solvents capable of dissolving hydrogen chloride and being miscible with water, exclusive of tetrahydrofuran. For example, such solvents may be lower alkyl alcohols such as methanol, ethanol or propanol, lower alkylnitriles such as acetonitrile or propionitrile, or dimethylformamide, and a mixed solvent thereof, preferably a lower alcohol, more preferably methanol.

The concentration of the hydrogen chloride solution or hydrochloric acid is generally, but not limited to, 0.01% to that of a saturated solution.

Hydrogen chloride, the hydrogen chloride solution and hydrochloric acidmay be added at a temperature which is generally, but not limited to, 0 to 100°C.

Hydrogen chloride, the hydrogen chloride solution or hydrochloric acid may be added at any rate without limit, depending on the amount to be manufactured. In order to prevent mixing of different crystalline forms resulting from abrupt supersaturation, however, instant addition should be avoided, and the addition should generally be performed over 2 minutes or more, preferably 15 minutes or more.

A supersaturated solution of the dihydrochloride of Compound (I) may also be prepared by a method of concentrating a dihydrochloride hydrate solution. For example, the concentration method may be a method of concentrating the solution by evaporating the solvent by heating under normal or reduced pressure with a rotary evaporator or the like, or a method of concentrating the solution with a reverse osmosis membrane. For example, the reverse osmosis membrane for use in the concentration of the solution may be selected from a polyacrylonitrile membrane, a polyvinyl alcohol membrane, a polyamide membrane, a cellulose acetate membrane, and the like.

The temperature for the crystallization of hydrate Crystalline form A of the dihydrochloride of Compound (I) according to the invention is generally from -70 to 150°C, preferably from -70 to 100°C.

While crystals may spontaneously start to precipitate in the reaction vessel, precipitation of the crystals may be started or promoted by adding a seed crystal or applying ultrasonic stimulation or mechanical stimulation such as by scratching the surface of the reaction vessel.

When a seed crystal is used for the crystallization of Crystalline form A, the seed crystal is not particularly restricted as long as it is a dihydrochloride hydrate crystalline form. In a preferred mode, it is the Crystalline form A dihydrochloride hydrate of Compound (I) according to the invention.

The amount of the seed crystal of the dihydrochloride hydrate is preferably, but not limited to, 0.001 to 20% by weight, more preferably 0.01 to 10% by weight, based on the weight of Compound (I) used or any of various salts thereof, or any of various solvates thereof.

Crystalline form B of the invention, which is a monohydrate crystalline form of the dihydrochloride of Compound (I), may be manufactured by a process including: dissolving Compound (I) or a salt of Compound (I) or any of various solvates thereof, the dihydrochloride of Compound (I) or any of various solvates thereof, or the Compound (I) dihydrochloride hydrate itself in a solvent; subjecting the solution to desalting (neutralization) ; adding hydrogen chloride or hydrochloric acid to the solution; bringing the solution into a supersaturated state by concentrating or cooling the solution or mixing of good and poor solvents or any other method to precipitate the dihydrochloride hydrate of Compound (I); and isolating the precipitated crystalline form.

A solution or suspension of a Compound (I)-containing crude product of a synthetic reaction in a solvent other than tetrahydrofuran may be used as a starting material for the manufacture of Crystalline form B, because Compound (I) in such a solution or suspension can be purified by the crystallization.

Since Crystalline form B is a hydrate crystalline form, the manufacture of Crystalline form B requires water. Therefore, the solvent for use in the manufacture of Crystalline form B should be only water or a water-miscible solvent other than tetrahydrofuran.

For example, such a water-miscible solvent may be a lower alkyl alcohol such as methanol, ethanol or propanol, a lower alkylnitrile such as acetonitrile or propionitrile, dimethylformamide, or a mixed solvent thereof, preferably a lower alcohol, more preferably methanol.

When a salt of Compound (I) or any of various solvates thereof is used as a starting material for the manufacture of Crystalline form B, desalting (neutralization) may be performed to ensure the production of the dihydrochloride of Compound (I). In such a case, an acid or a base is generally used for the desalting. The acid or base to be used may be any acid or base with which the desalting is possible. Examples of such an acid or base that may be used include the acids or bases listed above for use in the desalting for the manufacture of the free-form crystalline form of the

invention. Such an acid or base is preferably a hydrogen halide, an inorganic acid, an alkali metal hydroxide, or an amine, more preferably hydrogen chloride, hydrogen bromide, sulfuric acid, lithium hydroxide, sodium hydroxide, potassium hydroxide, ammonia, or tributylamine.

The acid or the base may be added as it is or in the form of a solution in any of various solvents.

The amount of the acid or base to be added is generally, but not limited to, 1 to 4 equivalents, based on the amount of any of various salts of Compound (I).

Crystalline form B may also be manufactured by adding hydrogen chloride or hydrochloric acid dropwise to a suspension of the free-form crystalline form of the invention.

The suspension of the free-form crystalline form of the invention to be used may be prepared by suspending the isolated free-form crystalline form of the invention in water. Alternatively, a suspension obtained by desalting (neutralizing) a salt of Compound (I) or any of various solvates thereof may also be used. Such a suspension may be manufactured according to the method of manufacturing the free-form crystalline form of the invention.

When hydrogen chloride or hydrochloric acid is added for the crystallization of Crystalline form B, the amount of the addition is preferably equal to or more than the amount necessary for the formation of the dihydrochloride of Compound (I) and for the crystallization thereof. In general, it may also be added in an amount of 0.1 to 20 equivalents, preferably 2 to 10 equivalents, based on the amount of Compound (I), so that the solubility of a dihydrochloride hydrate of Compound (I) in the solvent can be reduced.

Hydrogen chloride may be added directly or in the form of a solution in any of various organic solvents. Such organic solvents may be any solvents capable of dissolving hydrogen chloride and being miscible with water, exclusive of tetrahydrofuran. Examples of such solvents include the organic solvents listed above for use in the addition of hydrogen chloride for the manufacture of Crystalline form A. A lower alcohol is preferred, and methanol is more preferred.

The concentration of the hydrogen chloride solution or hydrochloric acid is generally, but not limited to, 0.01% to that of a saturated solution.

Hydrogen chloride, the hydrogen chloride solution or hydrochloric acidmay be added at a temperature which is generally, but not limited to, 0 to 100°C.

Hydrogen chloride, the hydrogen chloride solution or hydrochloric acid may be added at any rate, depending on the amount to be manufactured. In order to prevent mixing of different crystalline forms resulting from abrupt supersaturation, however, instant addition should be avoided, and the addition should generally be performed over 2 minutes or more, preferably 15 minutes or more.

A supersaturated solution of the dihydrochloride of Compound (I) may also be prepared by a method of concentrating a dihydrochloride hydrate solution. For example, the concentration method may be the method described above for the concentration in the process of manufacturing Crystalline form A.

The temperature for the crystallization of Crystalline form B of the invention is generally from -70 to 150°C, preferably from -70 to 100°C.

While crystals may spontaneously start to precipitate in the reaction vessel, precipitation of the crystals may be started or promoted by adding a seed crystal or applying ultrasonic stimulation or mechanical stimulation such as by scratching the surface of the reaction vessel.

When a seed crystal is used for the crystallization of Crystalline form B of the invention, the seed crystal is not particularly restricted as long as it is a dihydrochloride hydrate crystalline form. In a preferred mode, it is the Crystalline form B of the invention.

The amount of the seed crystal of the dihydrochloride hydrate is preferably, but not limited to, 0.001 to 20% by weight, more preferably 0.01 to 10% by weight, based on the weight of Compound (I) used or any of various salts thereof, or any of various solvates thereof.

In an embodiment of the invention, the precipitated dihydrochloride monohydrate crystalline forms (Crystalline forms A and B) of Compound (I) may be typically isolated by filtration, centrifugation, decantation, or any other technique.

In an embodiment of the invention, the isolated dihydrochloride monohydrate crystalline forms (Crystalline forms A and B) of Compound (I) may be washed with an appropriate solvent, if necessary. Washing the crystalline forms of the invention may be performed, for example, using water, dilute hydrochloric acid, a lower alkyl alcohol such as methanol, ethanol or propanol, a lower alkylnitrile such as acetonitrile or propionitrile, tetrahydrofuran, dimethylformamide, or a mixed solvent thereof, preferably using water or dilute hydrochloric acid, more preferably using dilute hydrochloric acid.

When dilute hydrochloric acid is used, the concentration thereof is generally, but not limited to, 0.001 to 20%, preferably 0.001 to 10%, more preferably 0.005 to 5%.

The isolated free-form crystalline form, Crystalline form A or Crystalline form B is generally dried at 0 to 150°C, preferably at 20 to 90°C until the weight becomes almost constant. If necessary, the crystalline forms may be dried in the presence of a desiccant such as silica gel or calcium chloride or under reduced pressure. When the drying is performed under reduced pressure, the temperature and the pressure may be controlled so that drying can proceed without removal of the crystal water. In such a case, when the drying temperature is high, the pressure should be controlled to be relatively

high. For example, when Crystalline form B of the invention is dried, the drying may generally be performed under a pressure of 0.7 to 50 kPa, preferably 1.8 to 11 kPa, at a drying temperature of 50°C.

When the drying is performed without control of temperature or pressure so that the crystalline form is dried and dehydrated, moisture absorption may generally be performed at a temperature of 0 to 50°C and a relative humidity of 10 to 100%, preferably at a temperature of 10 to 40°C and a relative humidity of 20 to 100% until the weight becomes almost constant.

The purity and quality of the resulting crystalline forms may be improved by recrystallization or slurry purification.

Compound (I) and pharmacologically acceptable salts thereof (in particular, preferably, a hydrochloride thereof) exhibit a significant ability to activate peroxisome proliferator-activated receptor (PPAR) γ as disclosed in Japanese Patent No. 3488099 (WO 99/18081, U.S. PatentNo. 6432993, European Patent No. 1022272) (Patent Document 1), JP-A No. 2003-238406 (WO 03/053440) (Patent Document 2), JP-A No. 2004-083574 (WO 2004/000356) (Patent Document 3), JP-A No. 2005-162727 (WO 2004/083167) (Patent Document 4), and WO 2007/091622 (Patent Document 5).

In particular, WO 2007/091622 (Patent Document 5) discloses that Compound (I) and its hydrochloride are useful for anticancer pharmaceutical compositions for preventing or treating stomach cancer,colon cancer,lung cancer, breast cancer, pancreas cancer, kidney cancer, prostate cancer, medulloblastoma, rhabdomyosarcoma, Ewing's sarcoma, liposarcoma, multiple myeloma, or leukemia.

More specifically, Test Example lin WO 2007/091622 (Patent Document 5) discloses experimental data showing that the dihydrochloride of Compound (I) exhibited significant activity to inhibit the growth of any of cancer cells including human stomach cancer cells, human breast cancer cells, small cell lung cancer, pancreas cancer cells, prostate cancer cells, kidney cancer cells, medulloblastoma, human sarcoma cells (rhabdomyosarcoma, Ewing's sarcoma, liposarcoma), and multiple myeloma.

Test Example 2 in the same publication (Patent Document 5) also discloses experimental data showing that the dihydrochloride of Compound (I) significantly inhibits the growth-inhibiting activity of human leukemia cells.

Test Example 3 in WO 2007/091622 (Patent Document 5) further discloses that the dihydrochloride of Compound (I) exhibits significant antitumor activity in vivo against human colon cancer cell lines.

Test Example 4 in the same publication (Patent Document 5) further discloses that the use of the dihydrochloride of Compound (I) in combination with an epidermal growth factor receptor (EGFR) inhibitor shows synergistic activity to inhibit the growth of cancer cells.

Test Example 5 in the same publication (Patent Document 5) further discloses that the dihydrochloride of Compound (I) has antitumor activity against human non- small-cell lung cancer and that the administration of it in combination with an epidermal growth factor receptor (EGFR) inhibitor shows enhanced antitumor activity.

Test Example 6 in the same publication (Patent Document 5) further discloses that the use of the dihydrochloride of Compound (I) in combination with a vascular endothelial growth factor receptor (VEGFR) inhibitor or a Raf kinase inhibitor shows synergistic activity to inhibit the growth of cancer cells.

Test Example 7 in the same publication (Patent Document 5) further discloses that the dihydrochloride of Compound (I) has antitumor activity against human kidney cancer and that the administration of it in combination with a vascular endothelial growth factor receptor (VEGFR) inhibitor or a Raf kinase inhibitor shows enhanced antitumor activity.

Therefore, the crystalline form of the invention is useful as a pharmaceutical material, particularly, a PPAR γ activator, and is also useful for agents (anticancer pharmaceutical compositions) for treating or preventing various cancers as described above.

In addition, Japanese Patent No. 3488099 (WO 99/18081, U.S. Patent No. 6432993, European Patent No. 1022272) (Patent Document 1) discloses that Compound (I) and pharmacologically acceptable salts thereof exhibit a significant ability to activate peroxisome proliferator-activated receptor (PPAR) γ, have significant activity to ameliorate insulin resistance and to reduce blood sugar, and are also useful as agents for treating or preventing diabetes (in particular, type 2 diabetes). Therefore, the crystalline form of the invention is useful for pharmaceutical compositions for preventing or treating diabetes (in particular, type 2 diabetes).

The crystalline form of the invention is also useful for pharmaceutical compositions for preventing or treating cancer occurring with type 2 diabetes, because it is useful for anticancer pharmaceutical compositions as described above.

In cases where the crystalline form of the invention is used as a medicine, particularly as a PPAR γ activator, an agent for treating or preventing cancer, or an agent for treating or preventing diabetes, the crystalline form may be administered by itself or mixed with an appropriate, pharmacologically-acceptable vehicle, diluent or any other material to form, for example, tablets, capsules, granules, powders, syrups, or other forms for oral administration, or to form, for example, injections, suppositories or other forms for parenteral administration.

These preparations are manufactured by known methods using additives such as vehicles (e.g., sugars such as lactose, sucrose, glucose, and sorbitol; corn starch, potato starch, α-starch, dextrin and starch derivatives such as

carboxymethyl starch; crystalline cellulose and cellulose derivatives such as hydroxypropylmethyl cellulose, carboxymethylcellulose, calcium carboxymethylcellulose, and sodium internally cross-linked carboxymethylcellulose; gum arabic; dextran; pullulan; silicates such as synthetic aluminum silicate and magnesium aluminometasilicate; phosphates such as calcium phosphate; carbonates such as calcium carbonate; and sulfates such as calcium sulfate), binders (e.g., the above vehicles; gelatin; polyvinylpyrrolidone; and macrogol), disintegrants (e.g., the above vehicles; chemically modified starch or cellulose derivatives such as croscarmellose sodium, carboxymethyl starch sodium, and cross-linked polyvinylpyrrolidone), lubricating agents (e.g., talc; stearic acid; metal stearates such as calcium stearate and magnesium stearate; colloidal silica; veegum; waxes such as beeswax and whale wax; boric acid; glycol; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acids such as silicic acid anhydride and silicic acid hydrates; the starch derivatives described above for the vehicles), stabilizers (e.g., p-hydroxybenzoates such as methylparaben and propylparaben; alcohols such as chlorobutanol, benzyl alcohol and phenylethyl alcohol; benzalkonium chloride; phenols such as phenol and cresol; thimerosal; acetic anhydride; and sorbic acid), corrigents (e.g., sweetening agents, souring agents and fragrant materials generally used), suspending agents (e.g., polysorbate 80 and sodium carboxymethylcellulose), diluents, and solvents for preparation (e.g., water, ethanol and glycerin).

The amount of the crystalline form of the invention to be used depends on the symptoms, body weight or age of the patient (a mammal, in particular a human) in need of administration, the mode of administration, or other conditions. For example, it is preferably administered in a dose of 0.001 mg/kg body weight (more preferably 0.01 mg/kg body weight) (as a lower limit) to 500 mg/kg body weight (more preferably 50 mg/kg body weight) (as an upper limit) for oral administration or in a dose of 0.005 mg/kg body weight (more preferably 0.05 mg/kg body weight) (as a lower limit) to 50 mg/kg body weight (more preferably 5 mg/kg body weight) (as an upper limit) for intravenous administration, once to several times per day, depending on symptoms.

Examples

The invention is more specifically described by the examples, test examples and preparation examples shown below.

Example 1 (Crystalline form A)

(1-1)

A hydrochloride (4.0 g) of 5-(4-{[6-(4-amino-3,5-dimethylphenoxy)-l-methyl-l-H-benzimi dazol-2-yl]methoxy} benzyl)-1,3-thiazolidine-2,4-dione, which was obtained by the same method as described in Example 8 of Japanese Patent No. 3488099, except that the hydrochloride was formed in aqueous tetrahydrofuran, was suspended in a mixture of tetrahydrofuran (40 mL) and water (12 mL) at room temperature under a nitrogen stream, and an aqueous 25% sodium hydroxide solution (2.4 g) was added dropwise thereto to form a solution. The resulting solution was added dropwise to a suspension of activated carbon (0.4 g) in tetrahydrofuran (12 mL), which was prepared under a nitrogen stream, and the mixture was stirred at the same temperature for 20 minutes. The activated carbon was filtered off and then washed with tetrahydrofuran (12 mL). The filtrate and the wash liquor were combined, and water (12 mL) was added thereto. A mixed solution of 38% hydrochloric acid (3.2 g) and tetrahydrofuran (12 mL) was added dropwise to the resulting solution. The reaction mixture was stirred for 45 minutes. The mixture was cooled to 0°C and then further stirred for 2 hours. The resulting crystals were filtered off and dried under about 80 Pa at 50°C for 12 hours. The crystals were allowed to stand in the air for 3 hours to give crystals (3.64 g) of 5-(4-{[6-(4-amino-3,5-dimethylphenoxy)-l-methyl-l-H-benzimi dazol-2-yl] methoxy}benzyl)-1,3-thiazolidine-2,4-dione dihydrochloride monohydrate (Crystalline form A).

(1-2)

Fig. 1 shows a diffraction pattern obtained by X-ray powder diffraction (CuKα, λ = 1.54 angstroms) of the crystalline form prepared in Example (1-1). Table 1 shows peaks with a relative intensity of 9 or more, when the intensity of the maximum peak in the diffraction pattern shown in Fig. 1 is normalized as 100. Each number in Fig. 1 corresponds to each peak number in Table 1.

Table 1

| Peak No. | 2θ | d value | Relative intensity | Peak No. | 2θ | d value | Relative intensity |
|---|---|---|---|---|---|---|---|
| 1 | 8.08 | 10.93 | 27 | 17 | 22.60 | 3.93 | 29 |
| 2 | 8.48 | 10.42 | 11 | 18 | 22.92 | 3.88 | 33 |
| 3 | 12.36 | 7.16 | 44 | 19 | 23.36 | 3.84 | 20 |
| 4 | 12.64 | 7.00 | 14 | 20 | 23.72 | 3.75 | 12 |
| 5 | 15.16 | 5.84 | 59 | 21 | 24.34 | 3.65 | 36 |
| 6 | 15.78 | 5.61 | 34 | 22 | 25.48 | 3.49 | 100 |
| 7 | 16.16 | 5.48 | 53 | 23 | 26.00 | 3.42 | 10 |
| 8 | 17.50 | 5.06 | 13 | 24 | 27.54 | 3.24 | 17 |
| 9 | 18.90 | 4.69 | 26 | 25 | 28.08 | 3.18 | 10 |
| 10 | 19.16 | 4.63 | 14 | 26 | 28.68 | 3.11 | 11 |
| 11 | 19.76 | 4.49 | 19 | 27 | 29.08 | 3.07 | 10 |
| 12 | 20.14 | 4.41 | 29 | 28 | 30.00 | 2.98 | 23 |
| 13 | 20.56 | 4.32 | 23 | 29 | 30.58 | 2.92 | 15 |
| 14 | 21.28 | 4.17 | 39 | 30 | 31.68 | 2.82 | 13 |
| 15 | 21.96 | 4.04 | 20 | 31 | 32.66 | 2.74 | 11 |
| 16 | 22.20 | 4.00 | 16 | 32 | 34.62 | 2.59 | 9 |

Among these peaks, peaks at interplanar spacing d values of 10.93, 7.16, 5.84, 4.41, 3.65, and 3.49 angstroms are particularly characteristic of Crystalline form A.

Example 2

(2-1)

Crystalline form A (2.0 g) obtained by the same method as in Example (1-1) was suspended in water (40 mL) and stirred at 80°C for 20 minutes. A mixture of 38% hydrochloric acid (1.1 g) and water (8.4 mL) was added dropwise thereto at the same temperature over 5 minutes, and the mixture was stirred for 1 hour. After the mixture was cooled to 40°C, crystals were filtered off and washed with water (6 mL) to give wet crystals of 5-(4-{[6-(4-amino-3,5-dimethylphenoxy)-1-methyl-1-H-benzimi dazol-2-yl]methoxy}benzyl)-1,3-thiazolidine-2,4-dione dihydrochloride monohydrate. The resulting crystals were dried under about 80 Pa at 50°C for 14 hours. The crystals were allowed to stand in the air for 3 hours to give white crystals (1.83 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate.

(2-2) Crystalline form B

Crystals (5.0 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate obtained by the same method as in Example (2-1) were suspended in water (300 mL), and 38% hydrochloric acid (1.94 g) was added dropwise to the suspension and then stirred at 95°C to form a solution. A mixture of 38% hydrochloric acid (0.81 g) and water (5 mL) was added dropwise thereto at the same temperature, and crystals (0.25 g) obtained by the same method as in Example (2-1) were added thereto and stirred for 30 minutes. A mixture of 38% hydrochloric acid (6.14 g) and water (38 mL) was added thereto over 2 hours and further stirred for 30 minutes. After the mixture was cooled to 40°C, crystals were filtered off. The resulting crystals were washed with a mixture of 38% hydrochloric acid (0.39 g) and water (15 mL) and then dried under 4.3 kPa at 50°C for 16 hours to give crystals (5.0 g) of a dihydrochloride monohydrate (Crystalline form B).

(2-3)

Fig. 2 shows a diffraction pattern obtained by X-ray powder diffraction (CuKα, λ = 1.54 angstroms) of the

crystalline form prepared in Example (2-2). Table 2 shows peaks with a relative intensity of 10 or more, when the intensity of the maximum peak in the diffraction pattern shown in Fig. 2 is normalized as 100. Each number in Fig. 2 corresponds to each peak number in Table 2.

Table 2

| Peak No. | 2θ | d value | Relative intensity | Peak No. | 2θ | d value | Relative intensity |
|---|---|---|---|---|---|---|---|
| 1 | 8.12 | 10.88 | 42 | 19 | 22.20 | 4.00 | 13 |
| 2 | 8.54 | 10.34 | 16 | 20 | 22.32 | 3.98 | 22 |
| 3 | 12.42 | 7.12 | 42 | 21 | 22.44 | 3.96 | 32 |
| 4 | 12.66 | 6.99 | 15 | 22 | 22.78 | 3.90 | 40 |
| 5 | 14.92 | 5.93 | 15 | 23 | 23.08 | 3.85 | 24 |
| 6 | 15.20 | 5.82 | 81 | 24 | 23.36 | 3.80 | 26 |
| 7 | 15.90 | 5.57 | 26 | 25 | 24.18 | 3.68 | 52 |
| 8 | 16.24 | 5.45 | 54 | 26 | 24.48 | 3.63 | 19 |
| 9 | 17.52 | 5.06 | 21 | 27 | 24.90 | 3.57 | 12 |
| 10 | 18.86 | 4.70 | 18 | 28 | 25.48 | 3.49 | 100 |
| 11 | 19.08 | 4.65 | 11 | 29 | 25.82 | 3.45 | 12 |
| 12 | 19.30 | 4.60 | 15 | 30 | 27.50 | 3.24 | 23 |
| 13 | 19.86 | 4.67 | 24 | 31 | 29.62 | 3.01 | 18 |
| 14 | 20.04 | 4.43 | 34 | 32 | 30.02 | 2.97 | 35 |
| 15 | 20.48 | 4.33 | 26 | 33 | 31.66 | 2.82 | 16 |
| 16 | 21.26 | 4.18 | 36 | 34 | 33.04 | 2.71 | 14 |
| 17 | 21.92 | 4.05 | 18 | 35 | 35.46 | 2.53 | 12 |
| 18 | 22.04 | 4.03 | 11 | | | | |

Among these peaks, peaks at interplanar spacing d values of 10.88, 7.12, 5.82, 3.68, 3.49, 3.01, and 2.97 angstroms are main peaks particularly characteristic of Crystalline form B.

Example 3 (Free-Form Crystalline form)

(3-1)

Crystals (80.00 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate obtained by the same method as in Example (2-1) were suspended in water (4,800 mL) and stirred at 80°C for 1 hour. After the suspension was cooled to 25°C, crystals were filtered off and washed with water (20 mL). The resulting crystals were dried under about 4.3 kPa at 50°C for 13.5 hours to give crystals (64.80 g). The crystals (63.00 g) were suspended in water (3,780 mL) and stirred at 80°C for 1 hour. After the suspension was cooled to 25°C, crystals were filtered off and washed with water (189 mL). The resulting crystals were dried under about 4.3 kPa at 50°C for 18 hours to give crystals (62.07 g) of 5-(4-{[6-(4-amino-3,5-dimethylphenoxy)-1-methyl-1-H-benzimi dazol-2-yl]methoxy}benzyl)-1,3-thiazolidine-2,4-dione monohydrate (free-form crystalline form).

(3-2)

Fig. 3 shows a diffraction pattern obtained by X-ray powder diffraction (CuKα, λ = 1.54 angstroms) of the crystalline form prepared in Example (3-1). Table 3 shows peaks with a relative intensity of 7 or more, when the intensity of the maximum peak in the diffraction pattern shown in Fig. 3 is normalized as 100. Each number in Fig. 3 corresponds to each peak number in Table 3.

Table 3

| Peak No. | 2θ | d value | Relative intensity | Peak No. | 2θ | d value | Relative intensity |
|---|---|---|---|---|---|---|---|
| 1 | 11.40 | 7.73 | 10 | 15 | 23.80 | 3.74 | 29 |
| 2 | 14.84 | 5.96 | 23 | 16 | 24.04 | 3.70 | 44 |
| 3 | 15.24 | 5.81 | 100 | 17 | 24.86 | 3.58 | 10 |
| 4 | 15.82 | 5.60 | 20 | 18 | 25.00 | 3.56 | 11 |
| 5 | 18.44 | 4.81 | 7 | 19 | 25.28 | 3.52 | 8 |
| 6 | 18.78 | 4.72 | 9 | 20 | 26.02 | 3.42 | 8 |
| 7 | 19.16 | 4.63 | 14 | 21 | 26.36 | 3.38 | 7 |
| 8 | 19.36 | 4.58 | 16 | 22 | 27.72 | 3.22 | 8 |
| 9 | 19.98 | 4.44 | 29 | 23 | 29.20 | 3.06 | 10 |
| 10 | 20.20 | 4.39 | 17 | 24 | 29.38 | 3.04 | 10 |
| 11 | 20.74 | 4.28 | 14 | 25 | 29.58 | 3.02 | 8 |
| 12 | 21.32 | 4.16 | 35 | 26 | 30.14 | 2.96 | 8 |
| 13 | 22.34 | 3.98 | 12 | 27 | 30.64 | 2.92 | 7 |
| 14 | 23.80 | 3.91 | 8 | 28 | 31.30 | 2.86 | 7 |

Among these peaks, peaks at interplanar spacing d values of 5.81, 5.60, 4.44, 4.16, and 3.70 angstroms are main peaks particularly characteristic of the free-form crystalline form.

Example 4 (Crystalline form A)

A hydrochloride (7.0 g) of 5-(4-{[6-(4-amino-3,5-dimethylphenoxy)-l-methyl-l-H-benzimi dazol-2-yl]methoxy} benzyl)-1,3-thiazolidine-2,4-dione, which was obtained by the same method as described in Example 8 of Japanese Patent No. 3488099, except that the hydrochloride was formed in aqueous tetrahydrofuran, was suspended in a mixture of tetrahydrofuran (70 mL) and water (21 mL) at room temperature, and an aqueous 25% sodium hydroxide solution (4.2 g) was added dropwise thereto to form a solution. The resulting solution was added dropwise to a suspension of activated carbon (0.7 g) in tetrahydrofuran (35 mL), which was prepared under a nitrogen stream, and the mixture was stirred at the same temperature for 10 minutes. The activated carbon was filtered off and then washed with tetrahydrofuran (21 mL). The filtrate and the wash liquor were combined, and water (21 mL) was added thereto. A mixed solution of 38% hydrochloric acid (5.6 g) and tetrahydrofuran (21 mL) was added dropwise to the resulting solution. The reaction mixture was stirred for 45 minutes. The mixture was cooled to 0°C and then further stirred for 2 hours. The resulting crystals were filtered off and dried under about 80 Pa at 50°C for 12 hours. The crystals were allowed to stand in the air for 3 hours to give crystals (6.36 g) of 5-(4-{[6-(4-amino-3,5-dimethylphenoxy)-l-methyl-l-H-benzimi dazol-2-yl]methoxy} benzyl)-1,3-thiazolidine-2,4-dione dihydrochloride hydrate. The X-ray powder diffraction (CuKα, $\lambda$ = 1.54 angstroms) pattern of the resulting crystalline form and the diffraction pattern of Crystalline form A obtained in Example (1-1) were slightly different in diffraction intensity but consistent in diffraction angle (2θ).

Example 5

(5-1) Free-Form Crystalline form

Crystals (10.0 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate obtained by the same method as in Example (2-1)were suspended in water(600mL),and the suspension was stirred at 80°C for 1 hour. At that point, part of the precipitated crystals were sampled and subjected to X-ray powder diffraction measurement. The resulting diffraction pattern was the same as that of the free-form crystalline form obtained in Example (3-1).

(5-2) Crystalline form B

A mixture of 38% hydrochloric acid (18.6 g) and water (115 mL) was added dropwise at 65°C dropwise to the suspension prepared in Example (5-1) and stirred for 40 minutes. After the resulting mixture was cooled to 40°C, crystals were filtered off and washed with a mixture of 38% hydrochloric acid (0.78 g) and water (30 mL). The resulting crystals were dried under about 4.3 kPa at 50°C for 11.5 hours to give crystals (8.73 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate. The X-ray powder diffraction (CuKα, λ = 1.54 angstroms) pattern of the resulting crystalline form and the diffraction pattern of Crystalline form B obtained in Example (2-2) wereslightlydifferentindiffractionintensity but consistent in diffraction angle (2θ).

Example 6

(6-1) Free-Form Crystalline form

Crystalline form A (4.0 g) of 5-(4-{[6-(4-amino-3,5-dimethylphenoxy)-l-methyl-l-H-benzimi dazol-2-yl]methoxy} benzyl)-1,3-thiazolidine-2,4-dione dihydrochloride monohydrate obtained by the same method as in Example (1-1) were suspended in water (160 mL), and an aqueous 25% sodium hydroxide solution (1.08 g) was added dropwise to the suspension at 80°C and stirred for 1 hour. In this state, part of the suspended crystals were sampled and subjected to X-ray powder diffraction measurement. The resulting diffraction pattern was the same as that of the free-form crystalline form obtained in Example (3-1).

(6-2) Crystalline form B

After the suspension prepared in Example (6-1) was cooled to 65°C, a solution prepared by diluting 38% hydrochloric acid (0. 65 g) with water (4.0mL) was added dropwise to the suspension. Crystals (0. 02 g) obtained by the same method as in Example (2-1) was added to the suspension, and a solution prepared by diluting 38% hydrochloric acid (5.11 g) with water (31.6 mL) was added dropwise thereto at the same temperature over 1 hour. After further stirring for 30 minutes, crystals were filtered off and washed with a solution prepared by diluting 38% hydrochloric acid (0.31 g) with water (12 mL). The resulting crystals were dried under 4.3 kPa at 50°C for 17 hours to give crystals (3.98 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate. The X-ray powder diffraction (CuKα, λ = 1.54 angstroms) pattern of the resulting crystalline form and the diffraction pattern of Crystalline form B obtained in Example (2-2) wereslightlydifferentindiffractionintensity but consistent in diffraction angle (2θ).

Example 7

(7-1) Free-Form Crystalline form

Crystals (10.0 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate obtained by the same method as in Example (2-1) were suspended in water (350 mL), and a solution prepared by diluting an aqueous 25% sodium hydroxide solution (2.70 g) with water (50 mL) was added dropwise to the suspension at 80°C and stirred for 1 hour. In this state, part of the suspended crystals were sampled and subjected to X-ray powder diffraction measurement. The resulting diffraction pattern was the same as that of the free-form crystalline form obtained in Example (3-1).

(7-2) Crystalline form B

After the suspension obtained in Example (7-1) was cooled to 55°C, a solution prepared by diluting 38% hydrochloric acid (1.08 g) with water (3.0 mL) was added dropwise to the suspension over 1 hour. After a suspension of Crystalline form B (0.025 g) obtained by the same method as in Example (2-2) in a mixture of 38% hydrochloric acid (0.026 g) and water (1 mL) was added thereto, a solution prepared by diluting 38% hydrochloric acid (0.026 g) with water (1 mL) was further added dropwise thereto and stirred for 3 hours. A solution prepared by diluting 38% hydrochloric acid (11.87 g) with water (33 mL) was added thereto at the same temperature over 1 hour. After further stirring for 30 minutes, crystals were filtered off at the same temperature and washed with a solution prepared by diluting 38% hydrochloric acid (0.78 g) with water (30 mL). The resulting crystals were dried under 0.4 kPa at 50°C for 63 hours and then allowed to absorb moisture at room temperature for about 5 hours in a desiccator in which the humidity was controlled with an aqueous saturated magnesium nitrate solution (to a humidity of about 53%), so that crystals of (9.77 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate were obtained. The X-ray powder diffraction (CuKα, λ = 1.54 angstroms) pattern of the resulting crystalline form and the diffraction pattern of Crystalline form B obtained in Example (2-2) were slightly different in diffraction intensity but consistent in diffraction angle (2θ).

(7-3)

The result of the elemental analysis of the resulting crystalline form is shown below.
Calculated ($C_{27}H_{30}N_4O_5SCl_2$): C = 54.64%; H = 5.09%; N = 9.44%; O = 13.48%; Cl = 11.95%; S = 5.40%.
Found: C = 54.45%; H = 5.04%; N = 9. 45%; O=13.42%; Cl = 12.10%; S = 5.42%.

Example 8 (Crystalline form B)

A monohydrate (3.0 g) of 5-(4-{[6-(4-amino-3,5-dimethylphenoxy)-l-methyl-l-H-benzimi dazol-2-yl]methoxy} benzyl)-1,3-thiazolidine-2,4-dione obtained by the same method as in Example (3-1) was suspended in water (120 mL), and a mixture of 38% hydrochloric acid (0.81 g) and water (2. 3 mL) was added dropwise at 55°C to the suspension. A suspension of crystals (0.0075 g) obtained in the same method as in Example (2-1) in 9.6% hydrochloric acid (0.6 mL) was added thereto and stirred for 3 hours. A solution obtained by diluting 38% hydrochloric acid (3.73 g) with water (10.47 mL) was added dropwise thereto at the same temperature over 1 hour. After further stirring for 1 hour, crystals were filtered off and washed with a solution obtained by diluting 38% hydrochloric acid (0.23 g) with water (9 mL). The resulting crystals were dried under 4.3 kPa at 50°C for 61 hours to give crystals (3.29 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate. The X-ray powder diffraction (CuKα, λ = 1.54 angstroms) pattern of the resulting crystalline form and the diffraction pattern of Crystalline form B obtained in Example (2-2) were slightly different indiffraction intensity but consistent in diffraction angle (2θ).

Example 9 (Crystalline form B)

Methanol (2700 mL) was added to crystals (100 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate obtained by the same method as in Example (2-1). After the mixture was refluxed, 38% hydrochloric acid (16.17 g) was added thereto. The resulting solution was cooled to 50°C. After the insoluble matter was filtered off, water (440 mL) was added dropwise to the filtrate, while the interior temperature was kept at 55°C or higher. The reaction liquid was cooled to 5°C and stirred at 0 to 5°C for 1 hour, and the precipitated crystals were separated by filtration. The resulting crystals were dried under 4.3 kPa at 50°C for about 15 hours to give crystals (78.24 g) of 5-(4-{[6-(4-amino-3,5-dimethylphenoxy)-l-methyl-l-H-benzimi dazol-2-yl]methoxy}benzyl)-1,3-thiazolidine-2,4-dione dihydrochloride monohydrate. The X-ray powder diffraction (CuKα, λ = 1.54 angstroms) pattern of the resulting crystalline form and the diffraction pattern of Crystalline form B obtained in Example (2-2) wereslightlydifferentindiffractionintensity but consistent in diffraction angle (2θ).

Comparative Example 1

A hydrochloride of 5-(4-{[6-(4-amino-3,5-dimethylphenoxy)-l-methyl-l-H-benzimi dazol-2-yl]methoxy}benzyl)-1,3-thiazolidine-2,4-dione was manufactured by the method described in Example 8 of Japanese Patent No. 3488099. The resulting compound had a slight red-purple color. Fig. 4 shows a diffraction pattern obtained by X-ray powder diffraction (CuKα, λ = 1.54 angstroms) of the compound.

Comparative Example 2

Fig. 5 shows a diffraction pattern obtained by X-ray powder diffraction (CuKα, λ = 1.54 angstroms) of the crystalline form prepared in Example (2-1) of Example 2. Table 5 shows peaks with a relative intensity of 10 or more, when the intensity of the maximum peak in the diffraction pattern shown in Fig. 5 is normalized as 100. Each number in Fig. 5 corresponds to each peak number in Table 4.
Table 4

| Peak No. | 2θ | d value | Relative intensity | Peak No. | 2θ | d value | Relative intensity |
|---|---|---|---|---|---|---|---|
| 1 | 8.18 | 10.80 | 25 | 18 | 23.16 | 3.84 | 24 |

(continued)

| Peak No. | 2θ | d value | Relative intensity | Peak No. | 2θ | d value | Relative intensity |
|---|---|---|---|---|---|---|---|
| 2 | 8.58 | 10.30 | 11 | 19 | 23.48 | 3.79 | 17 |
| 3 | 12.52 | 7.06 | 100 | 20 | 24.14 | 3.68 | 40 |
| 4 | 14.90 | 5.94 | 13 | 21 | 24.62 | 3.61 | 18 |
| 5 | 14.98 | 5.91 | 14 | 22 | 25.12 | 3.54 | 16 |
| 6 | 15.28 | 5.79 | 46 | 23 | 25.54 | 3.48 | 76 |
| 7 | 16.02 | 5.53 | 28 | 24 | 27.50 | 3.24 | 19 |
| 8 | 16.32 | 5.43 | 43 | 25 | 28.72 | 3.10 | 14 |
| 9 | 17.56 | 5.05 | 14 | 26 | 28.98 | 3.08 | 14 |
| 10 | 18.88 | 4.70 | 24 | 27 | 29.74 | 3.00 | 17 |
| 11 | 19.38 | 4.58 | 11 | 28 | 30.06 | 2.97 | 31 |
| 12 | 20.00 | 4.44 | 38 | 29 | 30.68 | 2.91 | 17 |
| 13 | 20.48 | 4.33 | 25 | 30 | 31.76 | 2.82 | 10 |
| 14 | 21.24 | 4.18 | 30 | 31 | 32.80 | 2.73 | 12 |
| 15 | 21.94 | 4.05 | 17 | 32 | 33.20 | 2.70 | 14 |
| 16 | 22.36 | 3.97 | 34 | 33 | 35.50 | 2.53 | 11 |
| 17 | 22.72 | 3.91 | 42 | | | | |

Test Example 1 (Impurities)

The impurity contents of Crystalline form A obtained in Example 4, Crystalline form B obtained in Example (6-2) and the compound obtained in Comparative Example 1 were measured by the HPLC analysis method described below. As used herein, the term "impurity content" refers to the ratio that is obtained by integrating all peak areas measured under the measurement conditions shown below and each having a peak area ratio of 0.01% or more, subtracting, from the integrated peak area, the peak area of Compound (I) and the peak area detected when only the solvent is injected, and dividing the result of the subtraction by the peak area of Compound (I). The individual purities are each represented by the area ratio of each peak other than the peak of Compound (I) and the peak detected when only the solvent is injected, among the measured peaks with a peak area ratio of 0.01% or more.

An aqueous 0.01 mol/mL ammonium acetate solution was added to an aqueous 0.01 mol/mL acetic acid solution so that the pH was adjusted to 4.5. As a result, a 0.01 mol/mL ammonium acetate buffer was obtained. Water and acetonitrile were mixed in a volume ratio of 3:2 to form a sample solvent.

Precisely 0.01 g of the object to be measured was weighed and transferred to a 20 mL brown measuring flask. After about 1 mL of dimethyl sulfoxide was added thereto to form a solution, the solution was made up to 20 mL to form a sample solution. Precisely 1 mL of the sample solution was measured and transferred to a 100 mL brown measuring flask. The sample solvent was added thereto to form 100 mL of a standard solution.

Measurements were performed under the conditions below.

HPLC Measurement Conditions (1)

Detector: UV absorptiometer (measurement wavelength: 230 nm);
Column: XTerra $RP_{18}$ (4.6 mm x 150 mm) manufactured by Waters Corporation;
Column temperature: 40°C;
Mobile phase: 0.01 mol/mL ammonium acetate buffer-acetonitrile (65:35);
Flow rate: 1 mL/minute (under the present conditions, Compound (I) showed a retention time of approximately 25 minutes.);
Injection volume of the standard solution and the sample solution: 10 μL;
Area measurement range: 70 minutes after the start of the injection.

HPLC Measurement Conditions (2)

Detector: UV absorptiometer (measurement wavelength: 230 nm);
Column: XTerra $RP_{18}$ (4.6 mm x 150 mm) manufactured by Waters Corporation;
Column temperature: 40°C;
Mobile phase: 0.01 mol/mL ammonium acetate buffer-acetonitrile (56:44);
Flow rate: 1 ml/minute (under the present conditions, Compound (I) showed a retention time of approximately 8 minutes.);
Injection volume of the standard solution and the sample solution: 10 μL;
Area measurement range: 70 minutes after the peak eluting next to the peak at a relative retention time of 1.48 to Compound (I) .

The impurity content was calculated from the following formula:

Impurity content (%)
= [the sum of the individual impurities of 0.01% or more measured under HPLC Measurement Conditions (I)] + [the sum of the individual impurities of 0.01% or more measured under HPLC Measurement Conditions (2)],

wherein
[the sum of the individual impurities of 0.01% or more measured under HPLC Measurement Conditions (1)] (%) = $A_{i1}/A_{s1}$ ; and
[the sum of the individual impurities of 0.01% or more measured under HPLC Measurement Conditions (2)] (%) = $A_{i2}/A_{s2}$, wherein
$A_{s1}$: the peak area of Compound (I) in the standard solution measured under HPLC Measurement Conditions (1),
$A_{i1}$: the peak area of individual impurities of 0.01% or more measured under HPLC Measurement Conditions (1),
$A_{s2}$: the peak area of Compound (I) in the standard solution measured under HPLC Measurement Conditions (2), and
$A_{i2}$: the peak area of individual impurities of 0.01% or more measured under HPLC Measurement Conditions (2).
The measurement results are shown in Table 5.
Table 5

| Measurement method | Relative retention time [h] | Impurity (%) | | |
|---|---|---|---|---|
| | | Crystalline form obtained in Comparative Example 1 | Crystalline form A obtained in Example 4 | Crystalline form B obtained in Example (6-2) |
| HPLC measurement conditions (1) | 0.13 | 0.10 | - | - |
| | 0.189 | 0.14 | 0.04 | - |
| | 0.192 | 0.24 | | |
| | 0.22 | - | 0.07 | - |
| | 0.32 | - | 0.11 | 0.03 |
| | 0.35 | 0.51 | 0.06 | 0.12 |
| | 0.88 | 1.06 | - | - |
| | 1.15 | 1.57 | - | - |
| | 1.19 | 0.10 | - | 0.03 |
| | 1.71 | 0.10 | 0.06 | 0.06 |
| | 2.39 | - | 0.22 | 0.39 |
| | Others | 0.75 | 0.01 | 0.06 |
| HPLC measurement conditions (2) | 1.52 | - | 0.06 | 0.06 |
| | 2.06 | 0.18 | - | - |
| | 2.73 | - | 0.04 | 0.04 |
| | 3.58 | - | 0.03 | 0.04 |
| | 3.98 | 0.20 | 0.01 | 0.03 |
| | 6.19 | - | 0.04 | 0.06 |
| | Others | 0.30 | 0.02 | 0.03 |
| Impurity content (%) | | 5.25 | 0.77 | 0.95 |

It is apparent that the impurity contents of Crystalline form A and Crystalline form B according to the invention are very low.

Test Example 2 (Residual Solvent)

Residual solvents in Crystalline formB obtained in Example (6-2), the crystalline form obtained in Example (2-1) and the compound obtained in Comparative Example 1 were measured by gas chromatography according to the analysis method described below. (1) Method of preparing samples containing Crystalline form B and the crystalline form obtained in Example (2-1)

Dimethylformamide and water were mixed in a volume ratio of 7:3 to form a diluent liquid.

Precisely 1 mL of tert-butyl alcohol was measured, transferred to a 100 mL measuring flask, and dissolved with the diluent liquid to form 100 mL of a solution. Precisely 10 mL of the solution was measured and transferred to a 500 mL measuring flask, and the diluent liquid was added theretoto form 500 mL of a solution to form an internal standard solution. Precisely, 2 mL of tetrahydrofuran, 2 mL of diisopropyl ether, 2 mL of methanol, 2 mL of ethyl acetate, 2 mL of acetic acid, and 2 mL of 1,4-dioxane were measured and transferred to a 250 mL measuring flask. The internal standard solution was added thereto to form 250 mL of a solution. Precisely 1 mL of the solution was measured and transferred to a 100 mL measuring flask. The internal standard solution was added thereto to form 100 mL of a solution. Out of 100 mL of the resulting solution, 6 mL was precisely measured and transferred to a 20 mL headspace vial. The vial was plugged with a rubber stopper and tightly sealed with an aluminum cap by seaming, so that a standard solution was obtained. Precisely 0.1 g of the sample to be measured was weighed and transferred to a 20 mL headspace vial, and

6 mL of the internal standard solution was precisely added thereto. The vial was plugged with a rubber stopper and tightly sealed with an aluminum cap by seaming. The sample was completely dissolved by shaking in a water bath at 60 to 70°C to give a sample solution.

(2) Method of preparing a sample containing the compound of Comparative Example 1

Precisely 1 mL of tert-butyl alcohol was measured, transferred to a 100 mL measuring flask, and dissolved with chlorobenzene to form 100 mL of a solution. Precisely 10 mL of the solution was measured and transferred to a 500 mL measuring flask, and chlorobenzene was added thereto to form 500 mL of an internal standard solution. Precisely, 2 mL of tetrahydrofuran, 2 mL of diisopropyl ether, 2 mL of methanol, 2 mL of ethyl acetate, 2 mL of acetic acid, and 2 mL of 1,4-dioxane were measured and transferred to a 250 mL measuring flask. The internal standard solution was added thereto to form 250 mL of a solution. Precisely 1 mL of the solution was measured and transferred to a 100 mL measuring flask. The internal standard solution was added thereto to form 100 mL of a solution. Out of 100 mL of the resulting solution, 6 mL was precisely measured and transferred to a 20 mL headspace vial. The vial was plugged with a rubber stopper and tightly sealed with an aluminum cap by seaming, so that a standard solution was obtained. Precisely 0.1 g of the sample to be measured was weighed and transferred to a 20 mL headspace vial, and 6 mL of the internal standard solution and 100 μL of tributylamine were precisely added thereto. The vial was plugged with a rubber stopper and tightly sealed with an aluminum cap by seaming. The sample was completely dissolved by shaking in a water bath at 60 to 70°C to give a sample solution.

(3) Test Conditions

The amounts of residual solvents were measured under the following test conditions.

Detector: Hydrogen flame ionization detector;
Column: DB-624 (0.53 mm × 30 m) manufactured by J&W Inc.;
Column temperature: 40°C (heldfor5minutes) →temperaturerising (at a rate of 10°C/minute) →260°C (held for 3 minutes);
Temperature of sample vaporization chamber: 250°C;
Detector temperature: 300°C;
Carrier gas: Helium;
Column flow rate: 5 ml/minute (it was controlled so that the retention time of tetrahydrofuran was about 7 minutes.);
Split ratio: 1:10;
Sample injection method: split method;
Area measurement range: 20 minutes.

(4) Operating conditions for the headspace apparatus

Equilibrium temperature inside the vial (oven temperature): 85°C;
Equilibrium time inside the vial: 15 minutes;
Injection line temperature
Sample loop temperature: 95°C;
Transfer line temperature: 110°C;
Carrier gas: Helium;
Vial pressurization time: 0.20 minutes;
Vial pressurization pressure: about 10 kPa;
Sample loop fill time: 0.15 minutes;
Sample loop equilibrium time: 0.05 minutes;
Injection time: 1.0 minute;
Sample injection volume: 1 mL.

(5) Method for calculating residual solvents

The amount of each residual solvent is determined by the following formula.

$$\text{The amount (ppm) of each residual solvent} = (2 \times D \times Q_T \times 6 \times 10{,}000{,}000)/(Q_S \times 25{,}000 \times W),$$

wherein
W: the weight (g) of the sample;
D: the density (g/mL) of each solvent;
$Q_S$: the ratio of the peak area of each solvent in the standard solution to the peak area of the internal standard substance; and
$Q_T$: the ratio of the peak area of each solvent in the sample solution to the peak area of the internal standard substance.

The measurement results are shown in Table 6.

Table 6

| Solvent name | Residual amount (ppm) | | |
|---|---|---|---|
| | Compound obtained in Comparative Example 1 | Crystalline form obtained in Example (2-1) | Crystalline form B obtained in Example (6-2) |
| Methanol | 25 | ND | ND |
| Ethanol | 1713 | ND | ND |
| Hexane | ND | ND | ND |
| Diisopropyl ether | ND | ND | ND |
| Ethyl acetate | 9297 | ND | ND |
| Tetrahydrofuran | ND | 271 | 59 |
| Acetic acid | 3892 | ND | ND |
| 1,4-dioxane | ND | ND | ND |
| Total | 14926 | 271 | 59 |

The residual solvent amount is smaller in the crystalline form obtained in Example (2-1) than in the crystalline form obtained in Comparative Example 1. The crystalline form obtained in Example (2-1) and the crystalline form used as a starting material for the manufacture of Crystalline form B in Example (6-2) each correspond to Crystalline form A manufactured by the method described in Example (1-1). It is apparent that Crystalline form B has a smaller residual solvent amount, though it is a dihydrochloride monohydrate crystalline form similar to that obtained in Example (2-1). It is also apparent that the method of preparing Crystalline form B according to the invention is extremely effective in removing solvents.

Test Example 3 (Solubility in Dilute Hydrochloric Acid)

The free-form crystalline form manufactured by the process of Example 3, Crystalline form A manufactured by the process of Example 4, and the crystalline form manufactured by the process of Example (2-1) were measured for solubility in dilute hydrochloric acid by the method described below.

Concentrated hydrochloric acid was diluted with water to prepare dilute hydrochloric acid (with a pH of about 0.7 at 25°C) with a hydrochloric acid concentration of 9.6 mg/g, which was used as a test liquid. 500 mg of the crystalline form was weighed and added to 5 mL of the test liquid at 25°C and stirred at the same temperature. After 6 minutes, the resulting test liquid was sampled and filtered. The concentration of 5-(4-{[6-(4-amino-3,5-dimethylphenoxy)-l-methyl-l-H-benzimi dazol-2-yl]methoxy}benzyl)-l,3-thiazolidine-2,4-dione in the filtrate was determined.

The concentration of 5-(4-{[6-(4-amino-3,5-dimethylphenoxy)-l-methyl-l-H-benzimi dazol-2-yl]methoxy}benzyl)-l,3-thiazolidine-2,4-dione was determined by the method described below.

An aqueous 0.01 mol/mL ammonium acetate solution was added to an aqueous 0.01 mol/mL acetic acid solution so that the pH was adjusted to 4.5. As a result, a 0.01 mol/mL ammonium acetate buffer was obtained. Water, acetonitrile and methanol were mixed in a volume ratio of 55:40:5 to form a sample solvent.

In the sample solvent was dissolved 0.2 g of isoamyl 4-hydroxybenzoate so that 200 mL of an internal standard solution was obtained. About 0.04 g of a standard sample of 5-(4-{[6-(4-amino-3,5-dimethylphenoxy)-l-methyl-l-H-benzimi dazol-2-yl]methoxy}benzyl)-l,3-thiazolidine-2,4-dione dihydrochloride monohydrate was precisely weighed, transferred to a 200 mL measuring flask and dissolved in the sample solvent to form 200 mL of a solution. 5 mL of the solution was precisely measured and transferred to a 50 mL measuring flask. 10 mL of the internal standard solution was precisely added to the measuring flask, and the sample solvent was further added thereto to form 50 mL of a standard solution.
After sampling, the test liquid was filtered, and about 0.04 g of the filtered test liquid was precisely weighed and transferred to a 50 mL measuring flask. About 2.5 mL of dimethyl sulfoxidewasaddedthereto. After 10 mL of the internal standard solution was added thereto, the sample solvent was further added thereto to form 50 mL of a sample solution.
The concentration was measured under the test conditions below.

Detector: UV absorptiometer (measurement wavelength: 290 nm);
Column: Symmetry C18 (4.6 mm × 100 mm) manufactured by Waters Corporation;
Column temperature: 40°C;
Mobile phase: 0.01 mol/mL ammonium acetate buffer-acetonitrile (3:2);
Flow rate: 1 mL/minute (under the present conditions, 5-(4-{[6-(4-amino-3,5-dimethylphenoxy)-l-methyl-l-H-benzimi dazol-2-yl]methoxylbenzyl)-l,3-thiazolidine-2,4-dione showed a retention time of approximately 8 minutes.);
Injection volume of the standard solution and the sample solution: 10 μL;
Peak area measurement range: about 20 minutes after the start of the injection.

The concentration is calculated from the following formula.
The concentration (mg/g) of the test liquid = $1000 \times (Q_T \times W_S \times F_P \times 502.58)/(Q_S \times W_T \times 40 \times 593.52)$, wherein
$W_S$: the weight (g) of the standard sample when the standard solution is prepared;
$W_T$: the weight (g) of the test liquid when the sample solution is prepared;
Fp: the purity factor of the standard sample;
$Q_S$: the value obtained by dividing the peak area of the standard sample in the chromatogram of the standard solution by the peak area of the internal standard; and
$Q_T$: the value obtained by dividing the peak area of the sample in the chromatogram of the sample solution by the peak area of the internal standard.
The results are shown in Table 7.
Table 7

| | The concentration (mg/g) of 5-(4-{[6-(4-amino-3,5-dimethylphenoxy) -1-methyl-l-H-benzimidazol-2-yl] methoxy}benzyl)-1,3-thiazolidine-2,4-dione in dilute hydrochloric acid with a concentration of 9.6 mg/g at 25°C (mg/g) |
|---|---|
| Free-form crystalline form obtained in Example 3 | 0.579 |
| Crystalline form A manufactured according to Example 4 | 0.325 |
| Crystalline form manufactured according to Example (2-1) | 0.276 |

In dilute hydrochloric acid, Crystalline form A shows the highest concentration among the dihydrochloride monohydrate crystalline forms. The free-form crystalline form shows a higher concentration than all the dihydrochloride monohydrate crystalline forms. In conclusion, the free-form crystalline form and Crystalline formA are considered to show high solubility also in gastric acid having a similar acidity to that of dilute hydrochloric acid and are therefore expected to be highly absorbable.

Preparation Examples

Preparation Example 1 (Capsule)

After mixing 5 g of Crystalline form A obtained in Example 1, 115 g of lactose, 58 g of corn starch, and 2 g of

magnesium stearate using a V-type mixing machine, 180 mg of the mixture is charged into a No. 3 capsule to form a capsule.

Preparation Example 2 (Tablet)

After mixing 5 g of Crystalline form B obtained in Example 1, 90 g of lactose, 34 g of corn starch, 20 g of crystalline cellulose, and 1 g of magnesium stearate using a V-type mixing machine, 150 mg of the mixture is formed into a tablet using a tablet machine.

Preparation Example 3 (Suspension)

Methylcellulose is dispersed and dissolved in purified water to form a dispersion medium. Crystalline form B obtained in Example 1 is weighed in a mortar and kneaded while the dispersion medium is added in small amounts thereto. Purified water is added thereto to prepare 100 g of a suspension.

## Claims

1. A hydrate crystalline form of 5-(4-{[6-(4-amino-3,5-dimethylphenoxy)-l-methyl-l-H-benzimi dazol-2-yl]methoxy}benzyl)-l,3-thiazolidine-2,4-dione represented by Formula (I):

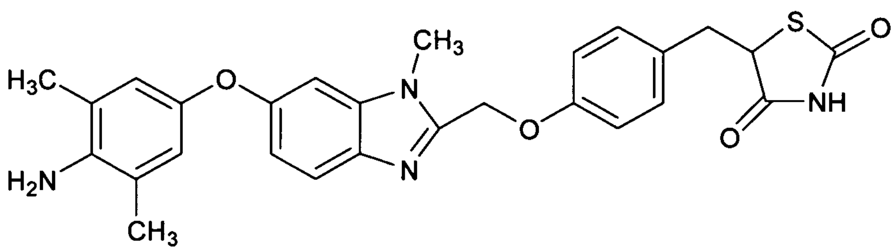

(I)

2. A monohydrate crystalline form of 5-(4-{[6-(4-amino-3,5-dimethylphenoxy)-l-methyl-l-H-benzimi dazol-2-yl]methoxy} benzyl)-l,3-thiazolidine-2,4-dione represented by Formula (I) shown in Claim 1, the crystalline form showing peaks at interplanar spacings of 5.81, 5.60, 4.44, 4.16, and 3.70 angstroms in powder X-ray diffraction obtained using copper K$\alpha$ radiation with a wavelength $\lambda$ of 1.54 angstroms.

3. A pharmaceutical composition comprising as an active ingredient the crystalline form of the thiazolidinedione compound represented by Formula (I) according to Claim 1 or 2.

4. A PPAR $\gamma$ activator comprising as an active ingredient the crystalline form of the thiazolidinedione compound represented by Formula (I) according to Claim 1 or 2.

5. An anticancer pharmaceutical composition comprising as an active ingredient the crystalline form of the thiazolidinedione compound represented by Formula (I) according to Claim 1 or 2.

6. A pharmaceutical composition for preventing or treating diabetes, comprising as an active ingredient the crystalline form of the thiazolidinedione compound represented by Formula (I) according to Claim 1 or 2.

7. A pharmaceutical composition for preventing or treating cancer occurring with type 2 diabetes, comprising as an active ingredient the crystalline form of the thiazolidinedione compound represented by Formula (I) according to Claim 1 or 2.

8. A method of manufacturing the crystalline formaccording to Claim 2, comprising:

desalting a salt of 5-(4-{[6-(4-amino-3,5-dimethylphenoxy)-l-methyl-l-H-benzimi dazol-2-yl]methoxylbenzyl)-l,3-thiazolidine-2,4-dione or a hydrate thereof in a water-containing solvent.

[Figure 1]

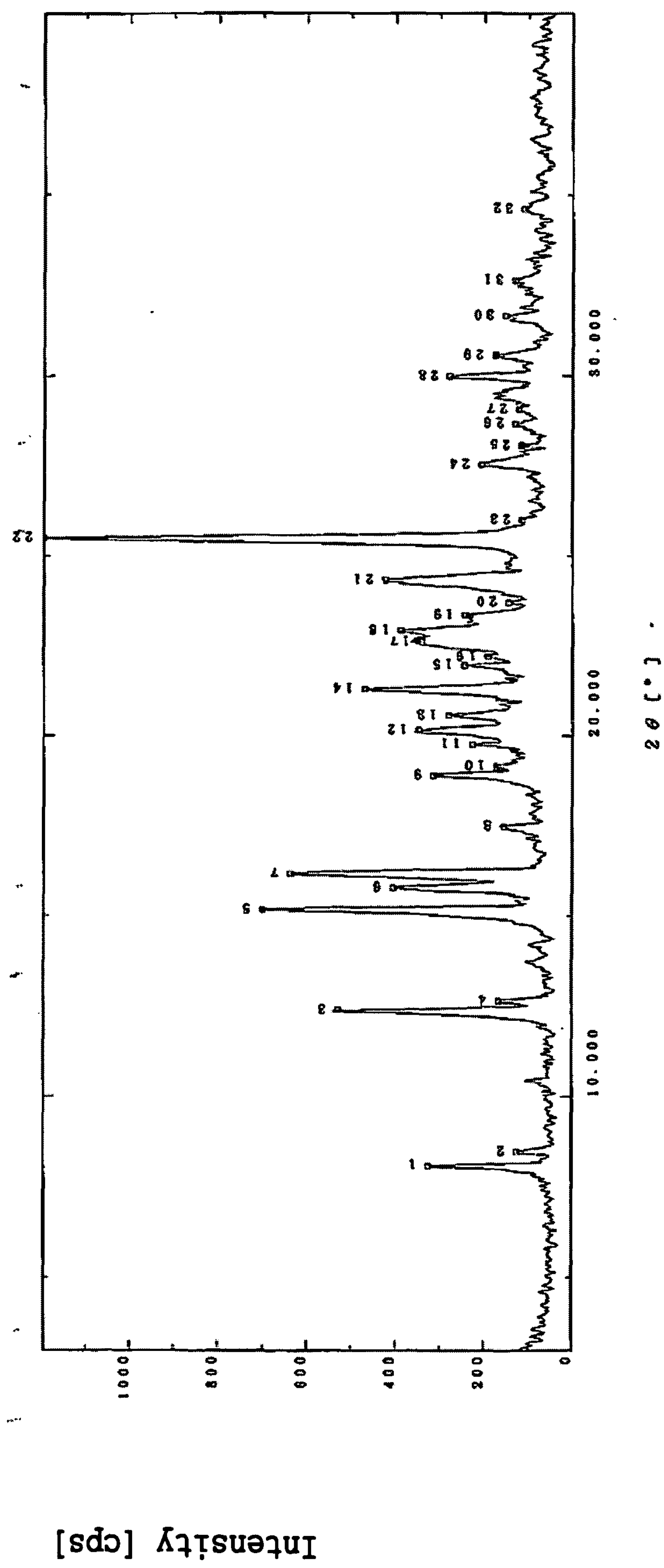

[Figure 2]

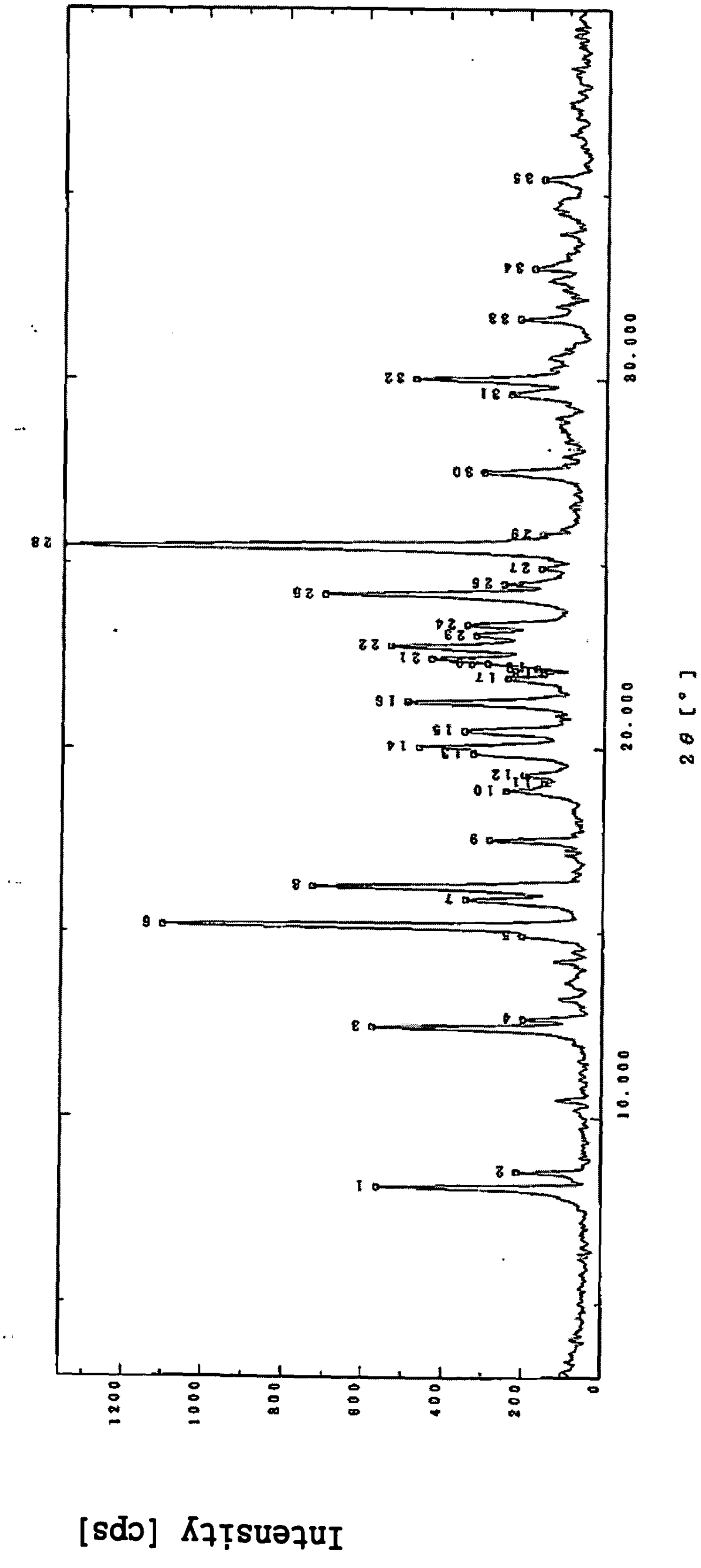
Intensity [cps]
2θ [°]
10.000
20.000
30.000
0
200
400
600
800
1000
1200

[Figure 3]

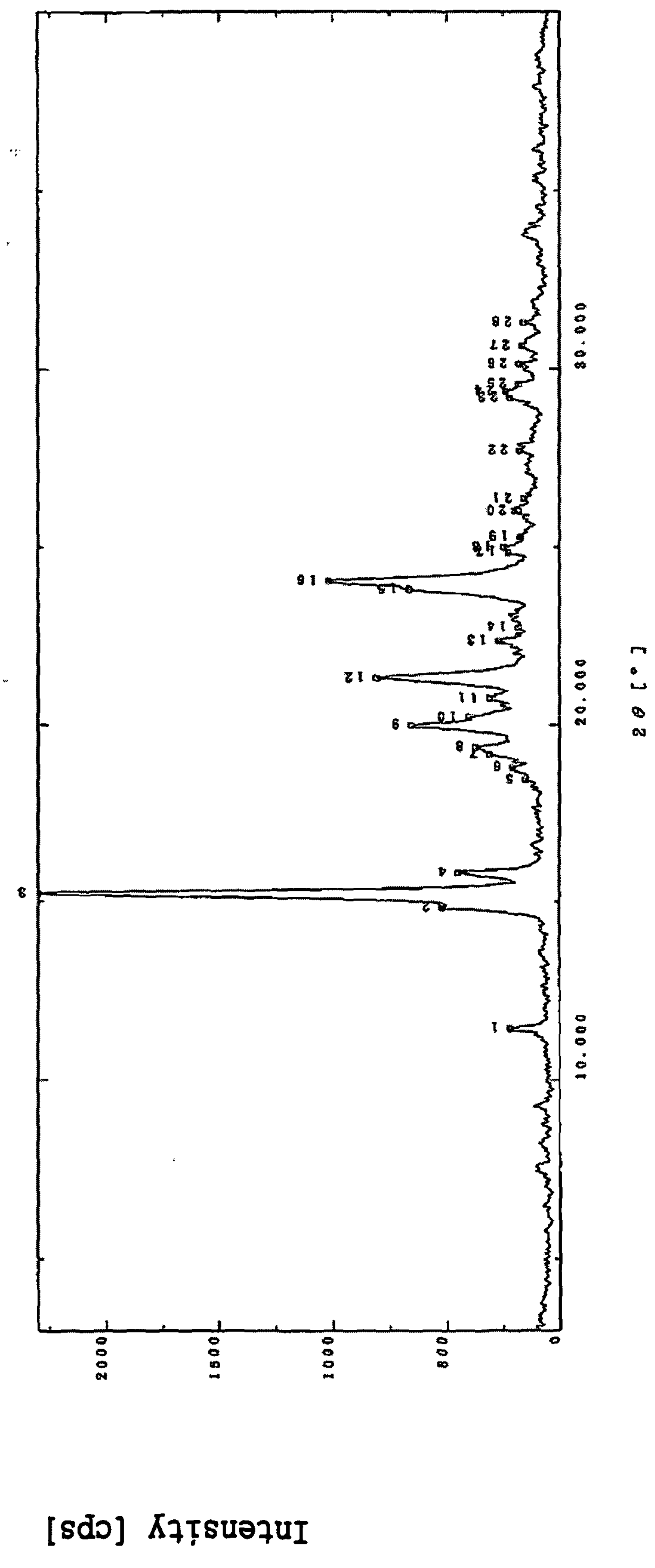

[Figure 4]

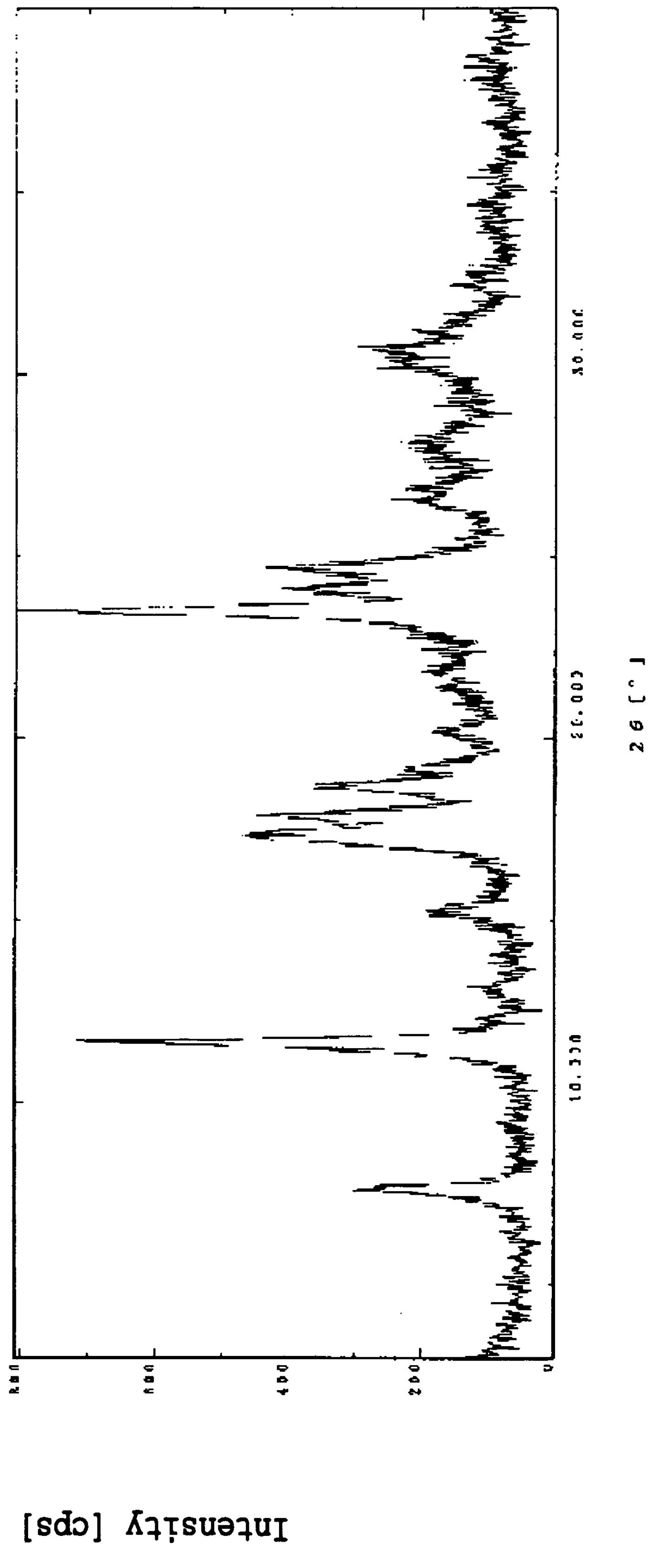

[Figure 5]

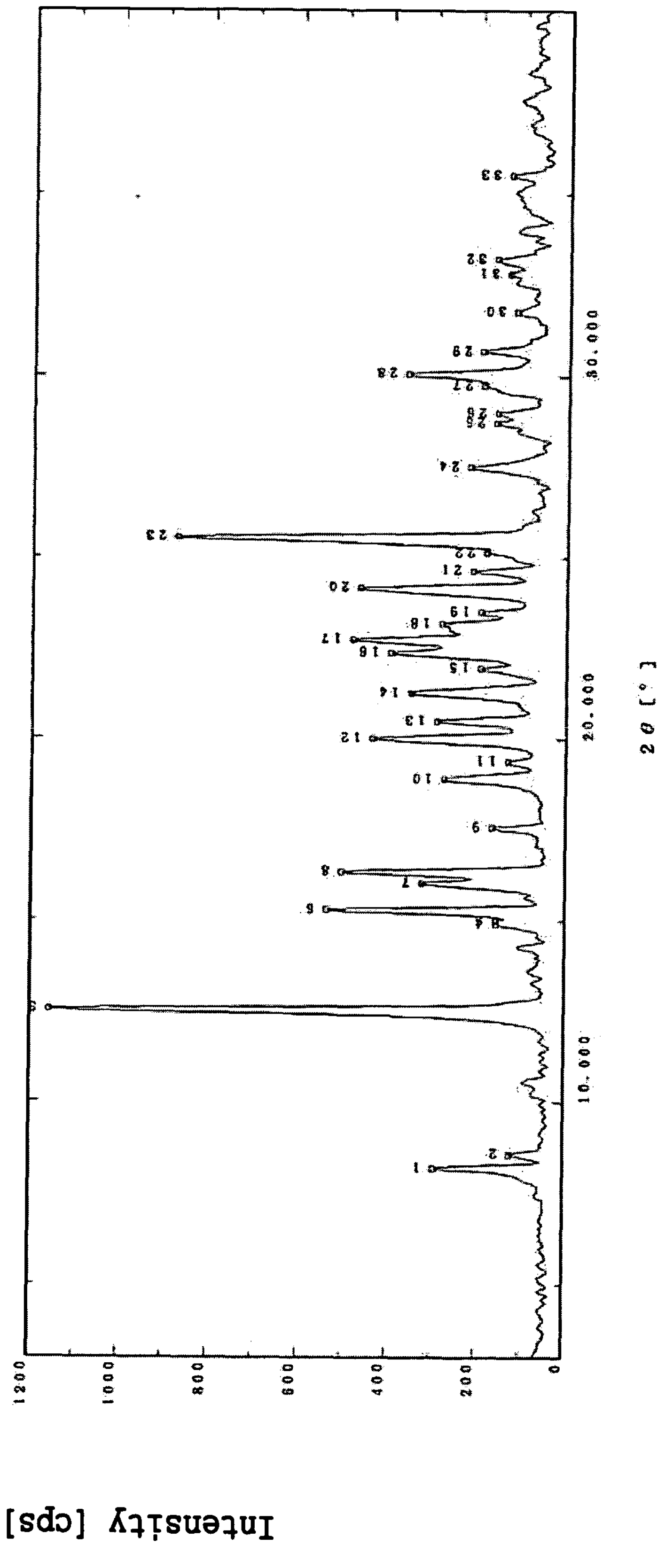
Intensity [cps]
0
200
400
600
800
1000
1200
10.000
20.000
30.000
2θ [°]

## INTERNATIONAL SEARCH REPORT

International application No.
PCT/JP2009/063558

A. CLASSIFICATION OF SUBJECT MATTER
C07D417/12(2006.01)i, A61K31/427(2006.01)i, A61P3/10(2006.01)i, A61P35/00 (2006.01)i, A61P43/00(2006.01)i, C07B63/00(2006.01)i

According to International Patent Classification (IPC) or to both national classification and IPC

B. FIELDS SEARCHED

Minimum documentation searched (classification system followed by classification symbols)
C07D417/12, A61K31/427, A61P3/10, A61P35/00, A61P43/00, C07B63/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2000-351779 A (SANKYO CO., LTD., JP), 19 December, 2000 (19.12.00), Par. Nos. [0331], [0332], [0080] & WO 2000/61581 A1 & EP 1167366 A1 & US 2003/078426 A1 & US 6562849 B1 & JP 4197387 B2 | 1-8 |
| X | JP 2002-179568 A (SANKYO CO., LTD.), 26 June, 2002 (26.06.02), Par. Nos. [0345], [0346], [0081] (Family: none) | 1-8 |
| X | JP 2000-351769 A (SANKYO CO., LTD.), 19 December, 2000 (19.12.00), Par. Nos. [0357] to [0359], [0053] & JP 4169450 B2 | 1-8 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 August, 2009 (19.08.09) | 01 September, 2009 (01.09.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/063558

C (Continuation). DOCUMENTS CONSIDERED TO BE RELEVANT

| Category* | Citation of document, with indication, where appropriate, of the relevant passages | Relevant to claim No. |
|---|---|---|
| X | JP 11-193276 A (SANKYO CO., LTD.), 21 July, 1999 (21.07.99), Par. Nos. [0300] to [0302], [0056] & WO 99/18081 A1 & EP 1022272 A1 & US 6432993 B1 & JP 3488099 B2 & EP 1022272 B1 | 1-8 |
| X | JP 2003-238406 A (SANKYO CO., LTD.), 27 August, 2003 (27.08.03), Par. Nos. [0116], [0034] & WO 2003/053440 A1 & AU 2002354460 A1 | 1-8 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3488099 B
- WO 9918081 A
- US 6432993 B
- EP 1022272 A
- JP 2003238406 A
- WO 03053440 A
- JP 2004083574 A
- WO 2004000356 A
- JP 2005162727 A
- WO 2004083167 A
- WO 2007091622 A